(19) Europäisches Patentamt / European Patent Office / Office européen des brevets

(11) **EP 3 899 546 B1**

(12) **EUROPEAN PATENT SPECIFICATION**

(45) Date of publication and mention
of the grant of the patent:
**16.08.2023 Bulletin 2023/33**

(21) Application number: **19821088.2**

(22) Date of filing: **18.12.2019**

(51) International Patent Classification (IPC):
**G01N 33/68** (2006.01)

(52) Cooperative Patent Classification (CPC):
**G01N 33/6896; G01N 2800/28; G01N 2800/325**

(86) International application number:
**PCT/EP2019/086068**

(87) International publication number:
**WO 2020/127566 (25.06.2020 Gazette 2020/26)**

(54) **MEANS AND METHOD FOR THE EARLY PREDICTION OF POOR NEUROLOGICAL OUTCOME IN OUT-OF-HOSPITAL CARDIAC ARREST SURVIVORS**

**MITTEL UND VERFAHREN ZUR FRÜHZEITIGEN VORHERSAGE EINES SCHLECHTEN NEUROLOGISCHEN ERGEBNISSES BEI ÜBERLEBENDEN EINES AUSSERKLINISCHEN HERZSTILLSTANDS**

**MOYENS ET PROCÉDÉS DE PRÉDICTION PRÉCOCE D'UN MAUVAIS RÉSULTAT NEUROLOGIQUE CHEZ LES SURVIVANTS D'UN ARRÊT CARDIAQUE EN DEHORS DE L'HÔPITAL**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.12.2018 EP 18214021**

(43) Date of publication of application:
**27.10.2021 Bulletin 2021/43**

(73) Proprietor: **Medizinische Universität Wien
1090 Wien (AT)**

(72) Inventors:
• **ADLBRECHT, Christopher
1190 Wien (AT)**
• **DISTELMAIER, Klaus
1180 Wien (AT)**
• **WURM, Raphael
1190 Wien (AT)**

(74) Representative: **Vossius & Partner
Patentanwälte Rechtsanwälte mbB
Siebertstraße 3
81675 München (DE)**

(56) References cited:
EP-A1- 3 020 333        WO-A1-2015/038069
US-A1- 2009 093 005

• HYO JIN KANG ET AL: "Structure of human [alpha]-enolase (hENO1), a multifunctional glycolytic enzyme", ACTA CRYSTALLOGRAPHICA SECTION D: BIOLOGICAL CRYSTALLOGRAPHY., vol. 64, no. 6, 1 June 2008 (2008-06-01), pages 651-657, XP055558600, DK ISSN: 0907-4449, DOI: 10.1107/S0907444908008561
• DISTELMAIER KLAUS ET AL: "Cardiac arrest does not affect survival in post-operative cardiovascular surgery patients undergoing extracorporeal membrane oxygenation", RESUSCITATION, ELSEVIER, IE, vol. 104, 21 April 2016 (2016-04-21), pages 24-27, XP029581062, ISSN: 0300-9572, DOI: 10.1016/J.RESUSCITATION.2016.03.028
• MARTIN ANNBORN ET AL: "The Combination of Biomarkers for Prognostication of Long-Term Outcome in Patients Treated with Mild Hypothermia After Out-of-Hospital Cardiac Arrest-A Pilot Study", THERAPEUTIC HYPOTHERMIA AND TEMPERATURE MANAGEMENT, vol. 6, no. 2, 1 June 2016 (2016-06-01), pages 85-90, XP055558652, ISSN: 2153-7658, DOI: 10.1089/ther.2015.0033

Remarks:
    The complete document including Reference Table(s) and the Sequence Listing(s) can be downloaded from the EPO website

**Description**

The present invention relates to a method for predicting the neurological outcome of a patient after cardiac arrest, the method comprising the step(s) of: (I) a) determining the level of alpha-enolase in a biological sample that has been

obtained from a patient after cardiac arrest; and b) comparing the level of alpha-enolase obtained in (a) with the median or mean level of alpha-enolase in biological samples from patients with positive neurological outcome after cardiac arrest, wherein said patient is diagnosed as having a negative neurological outcome if the alpha-enolase level is increased by at least 100% over the median or mean level of alpha-enolase, and/or a positive neurological outcome if the alpha-enolase level is below 150% as compared to the median or mean level of alpha-enolase; or (II) determining the level of alpha-enolase in a biological sample that has been obtained from a patient after cardiac arrest, wherein said patient is diagnosed as having a negative neurological outcome if the alpha-enolase level is above 50 $\mu$g/L, and/or a positive neurological outcome if the alpha-enolase level is below 50 $\mu$g/L.

Out-of-hospital cardiac arrest (OHCA) is a life-threatening event that calls for distinct, fast action by bystanders and the emergency medical services (Weaver WD, Cobb LA, Hallstrom AP, Fahrenbruch C, Copass MK, Ray R. Factors influencing survival after out-of-hospital cardiac arrest. J Am Coll Cardiol 1986;7:752-7). The annual incidence of OHCA in Europe and the United States is approximately 275,000 and 360,000 persons, respectively (Atwood C, Eisenberg MS, Herlitz J, Rea TD. Incidence of EMS-treated out-of-hospital cardiac arrest in Europe. Resuscitation 2005;67:75-80 and Benjamin EJ, Virani SS, Callaway CW et al. Heart Disease and Stroke Statistics-2018 Update: A Report From the American Heart Association. Circulation 2018;137:e67-e492). Depending on the region, only 10% of patients with OHCA survived for at least 30 days or to discharge from hospital, of these 8.7% with sustained cerebral disabilities due to hypoxic-ischaemic brain injury (Grasner JT, Lefering R, Koster RW et al. EuReCa ONE-27 Nations, ONE Europe, ONE Registry: A prospective one month analysis of out-of-hospital cardiac arrest outcomes in 27 countries in Europe. Resuscitation 2016;105:188-95). Because of optimized logistic and medical management, rates of primary survival increase and challenge caregivers with a rising numbers of patients with uncertain neurological outcome (Atwood C, Eisenberg MS, Herlitz J, Rea TD. Incidence of EMS-treated out-of-hospital cardiac arrest in Europe. Resuscitation 2005;67:75-80 and Rea TD, Eisenberg MS, Sinibaldi G, White RD. Incidence of EMS-treated out-of-hospital cardiac arrest in the United States. Resuscitation 2004;63:17-24).

Neurological consultation and multimodal prognostic assessment represents an integral part of post-resuscitation patient management and is of major clinical relevance for guidance of further treatment strategies. Clinical decisions about continuation, limitation or termination of intensive care therapy are essentially driven by the neurological assessment. In fact, active withdrawal of life sustaining treatment based on prognostication of a poor neurological outcome represents the most common cause of death in OHCA patients admitted to hospital (Lemiale V, Dumas F, Mongardon N et al. Intensive care unit mortality after cardiac arrest: the relative contribution of shock and brain injury in a large cohort. Intensive Care Med 2013;39:1972-80).

So far, accurate prediction of neurological outcome is highly limited due to the lack of consistent predictors of clinically relevant brain damage. For early neurological prognostication in comatose OHCA patients, a multimodal strategy is recommended, including clinical examination, neurophysiological tests, cerebral imaging and biomarkers (Nolan JP, Soar J, Cariou A et al. European Resuscitation Council and European Society of Intensive Care Medicine 2015 guidelines for post-resuscitation care. Intensive Care Med 2015;41:2039-56). Despite this multifaceted diagnostic approach, the predictive value of all these methods is limited and fateful decisions will be frequently postponed. This may lead to unnecessary prolongation of costly therapies and trembling uncertainty of the relatives (Wijdicks EF, Hijdra A, Young GB, Bassetti CL, Wiebe S. Practice parameter: prediction of outcome in comatose survivors after cardiopulmonary resuscitation (an evidence-based review): report of the Quality Standards Subcommittee of the American Academy of Neurology. Neurology 2006;67:203-10). In view of these far-reaching consequences not only for the patients, their relatives, and the clinical staff but also regarding its ethical and socioeconomic implications, there is an unquestionable great unmet need for well-established and highly reliable objective predictors (Guidelines 2000 for Cardiopulmonary Resuscitation and Emergency Cardiovascular Care. Part 2: ethical aspects of CPR and ECC. Circulation 2000;102:I12-21).

Based on continuous advances in proteomic profiling techniques to identify biomarker candidates and the establishment of multiple reaction monitoring (MRM) -mass spectrometry (MS) diagnostic assays for the quantitative measurement of selected candidates, proteomics research has been established as promising tool of plasma biomarker discovery and validation in cardiovascular medicine (Lam MP, Ping P, Murphy E. Proteomics Research in Cardiovascular Medicine and Biomarker Discovery. J Am Coll Cardiol 2016;68:2819-2830). It was hypothesized that specific brain-derived proteins, which are passively released upon cell damage or actively secreted into the blood stream as result of cerebral ischemia/damage, may be identified in patient plasma to reveal novel, more reliable prognostic information.

In summary, cardiac arrest is a devastating event. Despite improving resuscitation practices, mortality after an out-of-hospital cardiac arrest (OHCA) is still over 90% with many survivors being left with severe neurological impairment. On the other hand, some cardiac arrest patients do make a good recovery and return home to a meaningful quality of life. Accurately predicting those who will achieve a good neurological outcome in post-arrest comatose patients is difficult.

Therefore, there is an ongoing need for novel biomarkers that are suitable for the prediction of the neurological outcome of a patient after cardiac arrest. This need is addressed by the present invention.

The present invention therefore relates in a first aspect to a method for predicting the neurological outcome of a patient after cardiac arrest, the method comprising the steps of: a) determining the level of alpha-enolase in a biological sample that has been obtained from a patient after cardiac arrest; and b) comparing the level of alpha-enolase obtained in (a) with the median or mean level of alpha-enolase in biological samples from patients with positive neurological outcome after cardiac arrest, wherein said patient is diagnosed as having a negative neurological outcome if the alpha-enolase level is increased by at least 100% over the median or mean level of alpha-enolase, and/or a positive neurological outcome if the alpha-enolase level is below 150% as compared to the median or mean level of alpha-enolase.

The present invention relates in accordance with the first aspect also to a method for predicting the neurological outcome of a patient after cardiac arrest, the method comprising the step of determining the level of alpha-enolase in a biological sample that has been obtained from a patient after cardiac arrest, wherein said patient is diagnosed as having a negative neurological outcome if the alpha-enolase level is above 50 $\mu$g/L, and/or a positive neurological outcome if the alpha-enolase level is below 50 $\mu$g/L.

Cardiac arrest as used herein is a condition in which the heart (usually suddenly and unexpectedly) stops beating. If this happens, blood stops flowing to the brain and other vital organs. When the heart stops pumping blood, the brain is starved of oxygen. This causes the cardiac arrest patient to fall unconscious and to stop breathing. Cardiac arrest generally causes death if it is not treated within minutes. Cardiac arrest is preferably defined as the absence of both, spontaneous respiration and palpable pulse. Cardiac arrest is to be held distinct from a heart attack that happens when blood supplying the heart muscle is cut off due to a clot in one of the coronary arteries.

If the cardiac arrest is left untreated the stop of the blood flow to the brain upon cardiac arrest causes neurological damage since the neurons in the brain die from starvation of oxygen. The physical symptoms of neurological damage may include, for example, one or more of partial or complete paralysis, muscle weakness, partial or complete loss of sensation, seizures, difficulties in reading and writing, poor cognitive abilities, unexplained pain, and decreased alertness.

The "patient" or "subject" referred to herein is an animal or human, preferably human. The animal is preferably a domestic animal, such as dog, cat or horse.

In the claimed method the patient is preferably kept at mild induced hypothermia as soon as possible after the cardiac arrest; i.e. usually in the ambulance and hospital. Mild induced hypothermia is preferably a temperature in the range of 32°C-36°C. Keeping cardiac arrest patients at mild induced hypothermia provides a survival and neurological benefit (Donnino et al. (2015), Circulation; 132:2448-2456).

Alpha-enolase (also known as enolase 1 (ENO1)) is a glycolytic enzyme expressed in most tissues and is one of the isozymes of enolase. Each isoenzyme is a homodimer composed of 2 alpha, 2 gamma, or 2 beta subunits, and functions as a glycolytic enzyme. Alpha-enolase, in addition, functions as a structural lens protein (tau-crystallin) in the monomeric form. Alternative splicing of this gene results in a shorter isoform that has been shown to bind to the c-myc promoter and function as a tumor suppressor. Several pseudogenes have been identified, including one on the long arm of chromosome 1. Alpha-enolase has also been identified as an autoantigen in Hashimoto encephalopathy. As an enolase, ENO1 is a glycolytic enzyme that catalyzes the conversion of 2-phosphoglycerate to phosphoenolpyruvate. This isozyme is ubiquitously expressed in adult human tissues, including liver, brain, kidney, and spleen. Within cells, ENO1 predominantly localizes to the cytoplasm, though an alternatively translated form is localized to the nucleus. Its nuclear form, also known as MBP1, functions solely as a tumor suppressor by binding and inhibiting the c-myc protooncogene promoter, and lacks the glycolytic enzyme activity of the cytoplasmic form. ENO1 also plays a role in other functions, including its action as a cell surface receptor for plasminogen on pathogens such as streptococci and activated immune cells, leading to systemic infection or tissue invasion; an oxidative stress protein in endothelial cells; a lens crystalline; a heat shock protein; and a binding partner of cytoskeletal and chromatin structures to aid in transcription. Alpha-enolase is suggested to be involved in cellular stress, infections, autoantigen activities and the occurrence and metastasis of cancer in addition to its innate glycolytic function. It is preferred within the present invention that the alpha-enolase level that is determined relates to the level of alpha-enolase corresponding to protein database RCSB PDB entry 2PSN and as shown in SEQ ID NO: 1.

Means and methods for determining the alpha-enolase level in a biological sample are known in the art. The method to be used is not particularly limited as long as the alpha-enolase concentration and/or absolute amounts in the sample can be obtained. The alpha-enolase level may be a relative or absolute values. Relative values allow determining changes in levels of specific proteins between different samples that are analyzed using the same method. In case absolute amounts in the sample are measured the sample amount must either accord with the sample amounts from patients with positive neurological outcome or the sample amounts must be made comparable by adjustment, so that a 1:1 comparison of the absolute amounts can be done. Hence, absolute protein levels may be depicted, for example, as mass per unit volume (mass concentration) or as amount of substance per unit volume (molar concentration) and are method independent. Changes in relative or absolute protein levels may be expressed in the percentage changes or as fold changes, as will be further detailed herein below.

The alpha-enolase level may be determined on the mRNA level or the protein level, and is preferably determined on the basis of the protein level. Means and methods for determining the alpha-enolase protein level will be further detailed herein below.

The alpha-enolase mRNA level may be determined by (a) quantitative PCR, preferably quantitative real time PCR, or (b) a template/RNA amplification method followed by determining the expression level of alpha-enolase using a fluorescence- or luminescence-based quantification method.

In quantitative PCR (qPCR), the amount of amplified product is linked to fluorescence intensity using a fluorescent reporter molecule. The point at which the fluorescent signal is measured in order to calculate the initial template quantity can either be at the end of the reaction (endpoint semi-quantitative PCR) or while the amplification is still progressing (real-time qPCR).

In endpoint semi-quantitative PCR, fluorescence data are collected after the amplification reaction has been completed, usually after 30-40 cycles, and this final fluorescence is used to back-calculate the amount of template present prior to PCR.

The more sensitive and reproducible method of real-time qPCR measures the fluorescence at each cycle as the amplification progresses. This allows quantification of the template to be based on the fluorescence signal during the exponential phase of amplification, before limiting reagents, accumulation of inhibitors, or inactivation of the polymerase have started to have an effect on the efficiency of amplification. Fluorescence readings at these earlier cycles of the reaction will measure the amplified template quantity where the reaction is much more reproducible from sample to sample than at the endpoint.

A non-limiting example of a template/RNA amplification method followed by determining the expression level of the alpha-enolase mRNA using a fluorescence- or luminescence-based quantification method is a method combining transcription mediated amplification (TMA) and a hybridization protection assay (HPA). In more detail, such a method may comprise hybridizing one or more oligonucleotides ("capture oligonucleotides") that are complementary to alpha-enolase mRNA. The hybridized target sequences are then captured onto magnetic microparticles that are separated from the sample in a magnetic field. Wash steps may be utilized to remove extraneous components. Target amplification typically occurs via TMA, which is a transcription-based nucleic acid amplification method that utilizes two enzymes, Moloney murine leukemia virus (MMLV) reverse transcriptase and T7 RNA polymerase. A unique set of primers is used for alpha-enolase mRNA, preferably containing a promoter sequence for T7 RNA polymerase. T7 RNA polymerase produces multiple copies of RNA amplicon from the DNA copy. Detection of alpha-enolase mRNA level is achieved by HPA using single-stranded, chemiluminescent-labelled nucleic acid probes that are complementary to the one or more amplicons. Preferably, distinguishably labelled probes are used for each target amplicon. The labelled nucleic acid probes hybridize specifically to the amplicon. A "selection reagent" then differentiates between hybridized and unhybridized probes by inactivating the label on unhybridized probes. During the detection step, the chemiluminescent signal produced by the hybridized probe is measured in a luminometer and is reported as "Relative Light Units" (RLU), thereby quantifying the alpha-enolase mRNA level.

A biological sample as used herein describes a sample obtained from a patient. Biological samples encompass, e.g., a clinical sample. The source of the biological sample may be, but is not limited to, blood or any blood constituents, cerebrospinal fluid, brain tissue, striatal tissue, hippocampal tissue, periventricular white matter tissue, frontal cortical tissue, saliva, buccal cells, bone marrow, bronchoalveolar lavage, skin, hair, semen, feces, and an organ tissue, such as lung tissue, liver tissue and kidney tissue. The biological sample may be processed to contain compounds that are not naturally intermixed with the biological sample in nature, such as preservatives, anticoagulants, buffers, fixatives, nutrients, or antibiotics.

The mean or median level is preferably a median. Mean (or average) and median are both statistical terms that can be calculated to understand the central tendency of a set of statistical scores. The mean is the arithmetic average of a set of numbers, or distribution, whereas the median is the numeric value separating the higher half of a sample, a population, or a probability distribution, from the lower half. The median is more robust than the mean since the mean is more influenced by outliers. For normal distributions the values of the mean or median are usually close to each other.

With increasing preference the neurological outcome is predicted to be negative in the patient to be diagnosed if the level of alpha-enolase is increased at least 200%, at least 250%, at least 300%, at least 310%, at least 320%, at least 330%, at least 340% or at least 344% over the median or median level of alpha-enolase in patients with positive neurological outcome. Similarly, with increasing preference the neurological outcome is predicted to be positive in the patient to be diagnosed if the level of alpha-enolase is below 140%, below 130%, below 120%, below 110%, below 100%, below 75%, below 50% and below 31% of the mean or median level of alpha-enolase in patients with positive neurological outcome.

In this respect it is to be understood that the mean or median level of alpha-enolase in patients with positive neurological outcome is set to 100%. Hence, for example in case in the patient to be diagnosed the level of alpha-enolase is increased by 100% the level of alpha-enolase in this patient is twice as high as the mean or median level of alpha-enolase in patients with positive neurological outcome (i.e. a two-fold increase). For instance, if in the patient to be diagnosed the level of alpha-enolase is 100% of the level of the mean or median level of alpha-enolase in patients with positive neurological outcome, the two levels of alpha-enolase are the same.

An alpha-enolase level above the threshold level of 50 µg/L is capable to predict a negative neurological outcome with a specificity of 0.93 and a positive predictive value of 0.88. The specificity of a test relates to the test's ability to correctly reject healthy patients without a condition. The positive predictive value (PPV) is the probability that following a positive test result, that individual will truly have the diagnosed condition. The threshold level of alpha-enolase has been determined on the basis of a collection of samples from out of hospital resuscitated patients after cardiac arrest and their known neurological outcome.

Neurological outcome describes the degree of neurological recovery and cerebral performance of a patient after cardiac arrest. A negative neurological outcome is distinguished from a positive neurological outcome on the basis of whether the patient recovers from the cardiac arrest to an extent that allows an independent life (positive outcome) or not (negative outcome). An independent life shall be the ability to live independently and productively in the community and to live with the same or largely the same freedom of choice as a non-disabled person. This assessment is preferably done at the day when the patient is released from hospital or rehabilitation.

The inventors hypothesized without being bound by theory that brain-derived proteins, which are passively released upon cell damage or actively secreted into the body of the patient as result of cerebral ischemia/damage upon cardiac arrest may be identified in a sample of a patient after cardiac arrest. The inventors thus used advanced proteomic techniques to identify novel markers that improve prediction of neurological outcome after cardiac arrest.

It was surprisingly and unexpectedly found that determining the level of alpha-enolase alone and/or in combination with levels of further biomarkers, such as heat shock cognate 71 kDa protein, 14-3-3 protein zeta/delta and/or cofilin-1 allows more reliable and accurate prediction of neurological outcome of a patient after cardiac arrest. As shown in the appended examples, the invention thus provides an early prediction model for the positive or negative neurological outcome in cardiac arrest survivors.

It was also surprisingly and unexpectedly found that there is a significant improvement in neurological outcome prediction when alpha-enolase alone and/or together with heat shock cognate 71 kDa protein, 14-3-3 protein zeta/delta and/or cofilin-1 is/are included into a multifactor predictive model along traditional risk factors, in particular the prediction model as shown in Table 9 (Model 4).

Neurophysiological tests are recommended by current guidelines to be performed in individuals not regaining consciousness within two days after the event. However, since these tests are technically demanding, they are not readily available in the majority of hospitals. Early, reliable prognosis within the first days following return of spontaneous circulation (ROSC) is often impossible based on currently available models including clinical assessment and art-established biomarkers (e.g. NSE, S100). This leaves clinicians and relatives in uncertainty and the present invention is an important contribution to overcome this uncertainty.

In accordance with a preferred embodiment of the first aspect of the invention, the method comprises prior to step (a) the step of determining the median or mean level of alpha-enolase in biological samples from patients with positive neurological outcome after cardiac arrest.

Step (b) of the claimed method requires a comparison to the median or mean level of alpha-enolase in biological samples from patients with positive neurological outcome. In accordance with the above preferred embodiment the determination of this median or mean level of alpha-enolase forms part of the claimed method.

In this respect it is of note that it suffices to determine the median or mean level of alpha-enolase once in biological samples from patients with positive neurological outcome. Once the median or mean level of alpha-enolase has been determined the neurological outcome of a patient after cardiac arrest can be determined on the basis of the pre-determined median or mean level of alpha-enolase. For this reason, the step of determining the median or mean level of alpha-enolase in biological samples from patients with positive neurological outcome after cardiac arrest is only to be included into the method of the first aspect of the invention in accordance with the above preferred embodiment of the first aspect of the invention.

In accordance with a preferred embodiment of the first aspect of the invention, the method further comprises (I') c) determining the level of at least one additional biomarker in the biological sample obtained from a patient after cardiac arrest; and d) comparing the level of said at least one additional biomarker obtained in (c) with the median or mean level of said at least one additional biomarker in biological samples from patients with positive neurological outcome after cardiac arrest, wherein said at least one additional biomarker is selected from the group consisting of the heat shock cognate 71 kDa protein, 14-3-3 protein zeta/delta, and cofilin-1, and wherein said patient is diagnosed as having a negative neurological outcome if the level of said at least one additional biomarker is increased by at least 100% over the median or mean level of said at least one additional biomarker, and/or as having a positive neurological outcome if the level of said at least one additional biomarker is below 150% as compared to the median or mean level of said at least one additional biomarker.

In accordance with a further preferred embodiment of the first aspect of the invention, the method further comprises (II') determining the level of at least one additional biomarker in the biological sample obtained from a patient after cardiac arrest, wherein said at least one additional biomarker is selected from the group consisting of the heat shock cognate 71 kDa protein, 14-3-3 protein zeta/delta, and cofilin-1, and wherein said patient is diagnosed as having a negative neurological outcome if the heat shock cognate 71 kDa protein level is above 490 $\mu$g/L, the 14-3-3 protein zeta/delta protein level is above 2.7 mg/L and/or the cofilin protein level is above 180 $\mu$g/L, and/or a positive neurological outcome if the heat shock cognate 71 kDa protein level is below 490 $\mu$g/L, the 14-3-3 protein zeta/delta protein level is below 2.7 mg/L and/or the cofilin protein level is below 180 $\mu$g/L.

Also described herein is a method for predicting the neurological outcome of a patient after cardiac arrest, the method comprising the steps of: a) determining the level of at least one biomarker selected from the group consisting of alpha-enolase, the heat shock cognate 71 kDa protein, 14-3-3 protein zeta/delta, and cofilin-1 in a biological sample obtained from a patient after cardiac arrest; and b) comparing the level of said at least one biomarker obtained in (a) with the median or mean level of said at least one biomarker in biological samples from patients with positive neurological outcome after cardiac arrest, and wherein said patient is diagnosed as having a negative neurological outcome if the level of said at least one biomarker is increased by at least 100% over the median or mean level of said at least one biomarker, and/or as having a positive neurological outcome if the level of said at least one biomarker is below 150% as compared to the median or mean level of said at least one biomarker. The at least one biomarker is with increasing preference any at least two, any at least three and all four of alpha-enolase, the heat shock cognate 71 kDa protein, 14-3-3 protein zeta/delta, and cofilin-1.

Likewise described herein is a method for predicting the neurological outcome of a patient after cardiac arrest, the method comprising the step of determining the level of at least one biomarker selected from the group consisting of alpha-enolase, the heat shock cognate 71 kDa protein, 14-3-3 protein zeta/delta, and cofilin-1 in a biological sample obtained from a patient after cardiac arrest, wherein said patient is diagnosed as having a negative neurological outcome if the alpha-enolase level is above 50 $\mu$g/L, the heat shock cognate 71 kDa protein level is above 490 $\mu$g/L, the 14-3-3 protein zeta/delta protein level is above 2.7 mg/L and/or the cofilin-1 protein level is above 180 $\mu$g/L, and/or a positive neurological outcome if the alpha-enolase level is below 50 $\mu$g/L, the heat shock cognate 71 kDa protein level is below 490 $\mu$g/L, the 14-3-3 protein zeta/delta protein level is below 2.7 mg/L and/or the cofilin-1 protein level is below 180 $\mu$g/L

The heat shock cognate 71 kDa protein, also known as heat shock 70 kDa protein 8 or Hsc70 or Hsp73 is a heat shock protein of the Hsp70 family that in humans is encoded by the *HSPA8* gene on chromosome 11. Hsp70 proteins are part of a cellular chaperone network. They assist in the proper folding, stabilization and repair of proteins, such as the folding of newly translated proteins, the refolding of misfolded or aggregated proteins, or the membrane translocation of secretory proteins (Mayer, Bukau (2005) Cell Mol Life Sci 62(6):670-684). Hsp73 (or Hsc70), amongst other functions, binds to nascent polypeptides to facilitate correct folding and is involved in the disassembly of clathrin-coated vesicles (Tavaria et al. (1995) Genomics 29(1):266-268). It is preferred that the heat shock cognate 71 kDa protein level that is determined relates to the level of heat shock cognate 71 kDa protein corresponding to protein database RCSB PDB entry 1ATR and as shown in SEQ ID NO: 2.

14-3-3 protein zeta/delta belongs to a family of conserved regulatory molecules that are expressed in all eukaryotic cells. 14-3-3 proteins have the ability to bind a multitude of functionally diverse signaling proteins, including kinases, phosphatases, and transmembrane receptors. More than 200 signaling proteins have been reported as 14-3-3 ligands. It is an adapter protein implicated in the regulation of a large spectrum of both general and specialized signaling pathways. It binds to a large number of partners, usually by recognition of a phosphoserine or phosphothreonine motif. Binding generally results in the modulation of the activity of the binding partner. The protein 14-3-3 protein zeta/delta, belonging to the 14-3-3 protein family, is known to protect cells from numerous biological stress conditions. In particular, their role in cell survival by inhibiting apoptotic processes support their neuroprotective effects, as neuronal death is essentially triggered by apoptosis following cerebral ischemia. It is preferred that the 14-3-3 protein zeta/delta level that is determined relates to the level of 14-3-3 protein zeta/delta corresponding to protein database RCSB PDB entry 1QJB and as shown in SEQ ID NO: 3.

Cofilin-1 (non-muscle; n-cofilin), also known as CFL1, is a human gene, part of the ADF/cofilin family. Cofilin-1 is a widely distributed intracellular actin-modulating protein that binds and depolymerizes filamentous F-actin and inhibits the polymerization of monomeric G-actin in a pH-dependent manner. It is involved in the translocation of actincofilin complex from cytoplasm to nucleus. It is preferred that the cofilin-1 level that is determined relates to the level of cofilin-1 corresponding to protein database RCSB PDB entry 4BEX and as shown in SEQ ID NO: 4.

The levels of the heat shock cognate 71 kDa protein, 14-3-3 protein zeta/delta, and cofilin-1 including the mean or median levels thereof may be determined as is described herein above for the level(s) of alpha-enoalse. For example, also the levels of the heat shock cognate 71 kDa protein, 14-3-3 protein zeta/delta, and cofilin-1 may be determined on the basis of the mRNA or protein levesl, preferably on the basis of the protein levels. Further, also the mean or median levels are preferably median levels.

With increasing preference the neurological outcome is predicted to be negative in the patient to be diagnosed if the level of the heat shock cognate 71 kDa protein is increased by at least 200%, at least 225%, at least 250%, at least 260%, at least 270%, or at least 277% over the median or median level of the heat shock cognate 71 kDa protein in patients with positive neurological outcome. Similarly, with increasing preference the neurological outcome is predicted to be positive in the patient to be diagnosed if the level of the heat shock cognate 71 kDa protein is below 140%, below 130%, below 120%, below 110%, below 100%, below 75%, below 50% and below 26% of the mean or median level of the heat shock cognate 71 kDa protein in patients with positive neurological outcome.

With increasing preference the neurological outcome is predicted to be negative in the patient to be diagnosed if the level of 14-3-3 protein zeta/delta is increased by at least 200%, at least 250%, at least 300%, at least 350%, at least 360%, or at least 372% over the median or median level of 14-3-3 protein zeta/delta in patients with positive neurological outcome. Similarly, with increasing preference the neurological outcome is predicted to be positive in the patient to be diagnosed if the level of 14-3-3 protein zeta/delta is below 140%, below 130%, below 120%, below 110%, below 100%, below 75%, below 50% and below 24% of the mean or median level of 14-3-3 protein zeta/delta in patients with positive neurological outcome.

With increasing preference the neurological outcome is predicted to be negative in the patient to be diagnosed if the level of cofilin-1 is increased by at least 200%, at least 250%, at least 300%, at least 350%, at least 375%, at least 400%, at least 410% or at least 418% over the median or median level of cofilin-1 in patients with positive neurological outcome. Similarly, with increasing preference the neurological outcome is predicted to be positive in the patient to be diagnosed if the level of cofilin-1 is below 140%, below 130%, below 120%, below 110%, below 100%, below 75%, below 50% and below 18% of the mean or median level of cofilin-1 in patients with positive neurological outcome.

In accordance with a more preferred embodiment of the first aspect of the invention, the method further comprises prior to step (c) the step of determining the median or mean level of the at least one additional biomarker in biological samples from patients with positive neurological outcome after cardiac arrest.

Step (d) of the claimed method requires a comparison to the median or mean level(s) of the heat shock cognate 71 kDa protein, 14-3-3 protein zeta/delta, and/or cofilin-1 in biological samples from patients with positive neurological outcome. In accordance with the above preferred embodiment the determination of this/these median or mean level(s) of the heat shock cognate 71 kDa protein, 14-3-3 protein zeta/delta, and/or cofilin-1 forms part of the claimed method.

In this respect is of note that it suffices to determine the median or mean level(s) of alpha-enolase once in biological samples from patients with positive neurological outcome. Once the median or mean levels of the heat shock cognate 71 kDa protein, 14-3-3 protein zeta/delta, and/or cofilin-1 have been determined the neurological outcome of a patient after cardiac arrest can be determined on the basis of the pre-determined median or mean levels of the heat shock cognate 71 kDa protein, 14-3-3 protein zeta/delta, and/or cofilin-1. For this reason the step of determining the median or mean levels of the heat shock cognate 71 kDa protein, 14-3-3 protein zeta/delta, and/or cofilin-1 in biological samples from patients with positive neurological outcome after cardiac arrest is only to be included into the method of the first aspect of the invention in accordance with the above more preferred embodiment of the first aspect of the invention.

Heat shock cognate 71 kDa protein, 14-3-3 protein zeta/delta, and/or cofilin-1 level above the thresholds discussed herein above are also capable to predict a negative neurological outcome with high specificity and a high positive predictive value. These threshold levels have been determined on the basis of the collection of the same samples from out of hospital resuscitated patients after cardiac arrest on the basis of which the above-discussed alpha-enolase threshold level has been obtained.

In accordance with a preferred embodiment of the first aspect of the invention, the patients with positive neurological outcome after cardiac arrest are at least 5, preferably at least 10, more preferably at least 20, and most preferably at least 30 patients.

The method of the first aspect of the invention requires the use of a median or mean level of alpha-enolase, and, optionally, further the use of median or mean levels of the heat shock cognate 71 kDa protein, 14-3-3 protein zeta/delta, and/or cofilin-1 in biological samples from patients with positive neurological outcome. The meaning and calculation of the median or mean have been detailed herein above. As is apparent from this explanation, the higher the number of patients the more robust the median or mean level. It is therefore also contemplated herein to use at least 40, preferably at least 50, more preferably at least 75, and most preferably at least 100 patients.

In accordance with another preferred embodiment of the first aspect of the invention, the biological sample(s) is/are whole blood, plasma, serum, cerebrospinal fluid or brain tissue.

In this respect it is preferred that the sample of the patient to be diagnosed and the samples of the patients with positive neurological outcome are of the same kind, e.g. whole blood samples.

Within whole blood, plasma, serum, cerebrospinal fluid and brain tissue serum and brain tissue are preferred since these biological samples are used in the appended examples.

In accordance with a more preferred embodiment of the first aspect of the invention, the biological sample(s) is/are plasma.

Plasma is preferred over brain tissue since plasma samples can be obtained by minimally invasive obtaining a blood sample and then processing the blood to plasma.

In accordance with a further preferred embodiment of the first aspect of the invention, patients with a positive neurological outcome are patients having, after recovery from the cardiac arrest, a Cerebral Performance Category (CPC) score of 1 or 2.

Hence, a negative neurological outcome is preferably distinguished from a positive neurological outcome herein on the basis of the Cerebral Performance Category (CPC) score (Ajam ate al. (2011), Scand J Trauma Resusc Emerg Med; 19:38). The CPC score is widely used in research and quality assurance to assess the neurologic outcome following cardiac arrest. CPC score is divided in to the following five categories:

(1) Good cerebral performance: The patient has a normal life and is generally conscious, alert, able to work and has a normal life. The patient may have minor psychological or neurologic deficits (mild dysphasia, nonincapacitating hemiparesis, or minor cranial nerve abnormalities).

(2) Moderate cerebral disability: The patient is disabled but has an independent life. The patient is generally conscious, has sufficient cerebral function at least for part-time work in sheltered environment or independent activities of daily life (e.g. dress, travel by public transportation, food preparation). The patient may have hemiplegia, seizures, ataxia, dysarthria, dysphasia, or permanent memory or mental changes.

(3) Severe Cerebral Disability: The patient is conscious but disabled and dependent. In this respect conscious means being dependent on others for daily support (in an institution or at home with exceptional family effort) and/or having at least limited cognition. This category includes a wide range of cerebral abnormalities, from patients who are ambulatory but have severe memory disturbances or dementia precluding independent existence to those who are paralyzed and can communicate only with their eyes, as in the locked-in syndrome.

(4) Coma/Vegetative State: The patient is generally unconscious, unaware of surroundings and has no cognition. The patient also is generally not able to verbally or psychologically interact with the environment.

(5) Brain Death. The patient has a certified brain death or has been diagnosed as dead by traditional criteria.

The CPC is preferably determined in the art at the time of hospital discharge through hospital record review that involved only the specific hospitalization related to the resuscitation. In accordance with the above preferred embodiment a positive neurological outcome is a CPC 1 or 2. A negative neurological outcome is therefore preferably a CPC of 3 or greater. A negative neurological outcome is more preferably a CPC of 4 or 5.

In accordance with a yet further preferred embodiment of the first aspect of the invention, the level of alpha-enolase and optionally also the level of the at least one additional biomarker has been/have been/is/are determined by mass spectrometry, enzyme-linked immunosorbent assay (ELISA), electrochemiluminescence assay and/or a radioimmunoassay.

In accordance with all of mass spectrometry, enzyme-linked immunosorbent assay (ELISA), electrochemiluminescence assay and a radioimmunoassay protein levels of alpha-enolase and optionally also the level of the at least one additional biomarker selected from the heat shock cognate 71 kDa protein, 14-3-3 protein zeta/delta and cofilin-1 are determined.

Mass spectrometry as used herein refers to the application of mass spectrometry to study proteins. Mass spectrometry is an important method for the accurate mass determination and characterization of proteins, and a variety of methods and instrumentations have been developed for its many uses. The two primary methods used for the ionization of protein in mass spectrometry are electrospray ionization (ESI) and matrix-assisted laser desorption/ionization (MALDI). These ionization techniques are used in conjunction with mass analyzers such as tandem mass spectrometry. In general, the protein are analyzed either in a "top-down" approach in which proteins are analyzed intact, or a "bottom-up" approach in which protein are first digested into fragments.

The mass spectrometry is preferably liquid chromatography-mass spectrometry (LC-MS). LC-MS is an analytical chemistry technique that combines the physical separation capabilities of liquid chromatography (or HPLC) with the mass analysis capabilities of mass spectrometry (MS). Coupled chromatography - MS systems are advantageous because the individual capabilities of each technique interact synergistically. While liquid chromatography separates mixtures with multiple components, mass spectrometry provides structural identity of the individual components with high molecular specificity and detection sensitivity. This tandem technique can be used to analyze biochemical, organic, and inorganic compounds commonly found in complex samples of environmental and biological origin and is used herein to measure protein levels. For LC-MS, protein-containing samples may be digested with a protease and the resulting peptides may be separated by liquid chromatography and analyzed in a mass spectrometer. The mass-to-charge ratio of the analyzed peptides may allow identification and quantification of the respective proteins in the sample. This procedure may allow determining relative changes in protein levels of specific proteins between different samples. Alternatively, absolute protein abundances may be determined by LC-MS if reference peptides in a known concentration are available for calibration.

Enzyme-linked immunosorbent assay (ELISA) is a technique used to detect the presence of an antigen in a certain sample. The antigen is fixed to a surface, and then a specific antibody binds to this antigen. The antibody is linked to an enzyme, which converts the substrate to then be measured.

Electrochemiluminescence assays are used for analytical applications. The sample is incubated with (ruthenium labeled) antibody(s) to form a complex. After adding streptavidincoated microparticles, the complex is bound to solid phase. The microparticles are magnetically fixed to the electrode. Application of a voltage to the electrode then induces chemiluminescent emission which is measured and the results are determined via a calibration curve.

A radioimmunoassay is a technique for measurement of the concentration of peptides or proteins in biological fluids. It involves competitive binding of radio-labeled antigen and unlabeled antigen to a high-affinity antibody. The bound antigens are then separated from the unbound ones and the radioactivity is measured using a gamma counter.

In accordance with another preferred embodiment of the first aspect of the invention, the biological sample(s) obtained from patient(s) after cardiac arrest has/have been obtained within 96h, preferably within 72h, more preferably between 24 and 72h and most preferably about 48h after cardiac arrest.

In order to select the required treatment to minimize the negative health effects for a patient after cardiac arrest it is important to predicting the neurological outcome of a patient after cardiac arrest as early as possible. Accordingly also the biological sample(s) is/are to be obtained as early as possible and preferably within 7 days, more preferably within 96h, even more preferably within 72h and most preferably within about 48h.

Since the samples that were used in the appended examples were obtained 48h after cardiac arrest it is even more preferred to obtain the samples between 24 and 72h and most preferably about 48h.

The term "about" as used herein preferably refers to a deviation of $\pm 10\%$ and more preferably of $\pm 20\%$.

In accordance with a preferred embodiment of the first aspect of the invention, the method further comprises predicting the neurological outcome after cardiac arrest in the patient on the basis of one or more selected from the age of the patient, shockable rhythm, haemoglobin concentration, the time from cardiac arrest to the start of life support, the time from cardiopulmonary resuscitation to return of spontaneous circulation (ROSC), the maximum serum lactate within 24h after cardiac arrest, the maximum difference to the pH of 7.3 within 24h after cardiac arrest, and the level of the protein S-110B in the sample of the patient.

For all of the parameters age of the patient the patient, shockable rhythm, haemoglobin concentration, the time from cardiac arrest to the start of life support, the time from cardiopulmonary resuscitation to return of spontaneous circulation (ROSC), the maximum serum lactate within 24h after cardiac arrest, the maximum difference to the pH of 7.3 higher numbers of the respective parameters indicate a negative neurological outcome after cardiac arrest in the patient.

Within the patients that were examined in the appended examples, the patients with a negative neurological outcome had an average age of 61 while the patients with a positive neurological outcome had an average age of 53. It is therefore preferred that an age of 61 or more indicates a negative neurological outcome and/or an age of 53 or below indicates a positive neurological outcome

Within the patients that were examined in the appended examples, 21 patients (33,9%) with a negative neurological outcome had no shockable rhythm, while only 3 (9.7%) patients with a positive neurological outcome had no shockable rhythm. It is therefore preferred that a shockable rhythm indicates a positive neurological outcome and the absence of a shockable rhythm indicates a negative neurological outcome.

The time from the diagnosis of cardiac arrest to start of life support for the patients with a negative neurological outcome was on average $0.39 \pm 1.35$ min while it was $0.03 \pm 0.18$ min for the patients with a positive neurological outcome. It is therefore preferred that a time of $0.39 \pm 1.35$ min or more indicates a negative neurological outcome and/or a time of $0.03 \pm 0.18$ min or less indicates a positive neurological outcome.

The time from cardiopulmonary resuscitation to return of spontaneous circulation (ROSC) for the patients with a negative neurological outcome was on average 27.5 min while it was 26.5 min for the patients with a positive neurological outcome. It is therefore preferred that a time of 27.5 min or more indicates a negative neurological outcome and/or a time of 26.5 min or less indicates a positive neurological outcome

The maximum serum lactate within first 24h for the patients with a negative neurological outcome was on average 7.6 mmol/L while it was 6.9 mmol/L for the patients with a positive neurological outcome. It is therefore preferred that a concentration of 7.6 mmol/L or more indicates a negative neurological outcome and/or a concentration of 6.9 mmol/L or less indicates a positive neurological outcome.

The maximum absolute difference of pH to the pH of 7.3 within the first 24h for the patients with a negative neurological outcome was on average 0.2 while it was 0.17 for the patients with a positive neurological outcome. It is therefore preferred that a pH difference of 0.2 or more indicates a negative neurological outcome and/or a a pH difference of 0.17 or less indicates a positive neurological outcome.

Finally, the level of S-100B in the sample for the patients with a negative neurological outcome was on average 0.14 µg/L while it was 0.08 µg/L for the patients with a positive neurological outcome. It is therefore preferred that a concentration of 0.14 µg/L or more indicates a negative neurological outcome and/or a concentration of 0.08 µg/L or below indicates a positive neurological outcome.

The S100 proteins are a family of low-molecular-weight proteins found in vertebrates and characterized by two calcium-binding sites that have helix-loop-helix ("EF-hand type") conformation. Currently at least 21 different S100 proteins are known. S-100B is widely expressed in astrocytes, certain neuronal populations, Schwann cells, myeloid-derived cells, and a few other cell types. S-100B is a known outcome predictor after cardiac arrest (Wojtczak-Soska and Lelonek (2010), Cardiol J., 17(5):532-6.).

Described herein but not part of the invention is in a second aspect a method for treating a patient after cardiac arrest, the method comprising a) predicting the neurological outcome of said patient according to the method of the first aspect of the invention; and b) administering to the patient one or more agents for treating hypoxic brain damage, wherein said one or more agents is/are preferably selected from inhalative Xenon, sodium nitrite, cyclosporine A and/or 2-iminobiotin and/or an RNA.

The above definitions and preferred embodiments of the method of the first aspect of the invention are equally applicable to the method of the second aspect .

The second aspect as well as the herein below described third aspect not part of the invention require in addition to predicting the neurological outcome of a patient after cardiac arrest according to the method of the first aspect of the invention to administer agents or undertake other curative means for treating hypoxic brain damage in patients with a predicted negative neurological outcome and sometimes also in patients with a predicted positive neurological outcome. As such, provided herein but not part of the invention are treatment methods including steps to determine which kind of treatment a patient should receive after having had a cardiac arrest.

The methods of the first aspect of the invention reliably provide an indication of the most appropriate treatment to be used in cases where neurological outcome can, up to now, not reliably be determined. Thus, without the method of the first aspect of the invention, treatment options were chosen which did not necessarily appropriately reflect the likely neurological outcome of the patient. For example, a patient with a negative neurological outcome as determined by the methods of the invention may receive more aggressive treatment in order to increase likelihood of survival as compared to a patient with a higher likelihood of a positive neurological outcome even without such aggressive treatment. Thus, in particular for patients with a predicted negative neurological outcome, there exist a number of treatment options aimed at improving cerebral performance that are associated with a substantial risk of adverse events or are still in an experimental phase and should therefore only be used as a last resort.

These treatment options include but are not limited to inhalative Xenon (Laitio et al. (2016) JAMA 315(11):1120-8), sodium nitrite (Dezfulian et al. (2012) Nitric oxide 26(4):241-50), cyclosporine A for selected patients with early signs of multiple organ failure (Argaud et al. (2016) JAMA Cardiol. 1(5):557-65), and 2-Iminobiotin (i.e. an experimental selective neuronal and inducible nitric oxide synthase inhibitor) and RNA.

The RNA is preferably a microRNA, mRNAm, non-coding RNA, siRNA, shRNA or esiRNA.

For instance, the expression of the microRNA-140-5p elevates cerebral protection of dexmedetomidine against hypoxic-ischaemic brain damage (Han et al. (2017), J Cell Mol Med. 2018; 22:3167-3182). Moreover, the expression of the mRNA encoding histone deacytylase 1 prevents neuroinflammation in stroke patients (Wang et al. (2017), Stroke, 48(8):2211-2221). Similarly, the expression of the mRNA encoding the E2 ubiquitin ligase MDM 2 is correlated with better functional outcome after stroke (Rodriguez et al. (2018), 49(10):2437-2444). The expression of the mRNAs encoding molecular chaperones and heat shock proteins exerts an anti-apoptotic effect upon brain hypoxia (Hua et al. (2017), Neural Regen Res.;12(1):153-160).

While the expression of the above-discussed RNA molecules in a patient after cardiac arrest is expected to be beneficial for the patient, the expression of other RNA molecules is expected to be maleficial for the patient. For instance, the non-coding RNA H19 promotes neuroinflammation in stroke patients (Wang et al. (2017), Stroke, 48(8):2211-2221). Also the inhibition of the mRNAs encoding the Sprouty proteins, in particular Sprouty 2 reduces the secondary brain damage in neural lesion models and improves nerve regeneration (Hausott and Klimaschweski, Mol Neurobiol, 2018 Sep 17.). The inhibition of the mRNA encoding the casein kinase 2 (CK2) promotes axon function recovery upon stroke (Bastian et al (2018), Neurobiol Dis.. pii: S0969-9961(18)30149-9. doi: 10.10161j.nbd.2018.05.011, [Epub ahead of print]). Finally, the knock-down of the mRNA encoding micro-calpain increases the survival and protects hippocampal function after global brain ischemia (Bevers et al. (2010), Exp Neurol., 224(1):170-177).

Hence, depending on whether the expression of the RNA to be targeted exerts a beneficial or maleficial effect in the patient after cardiac arrest either (i) an RNA promoting the expression of the target RNA, or (ii) an RNA inhibiting the expression of the target RNA is to be used in connection with the second aspect

An RNA according to (i) may be a recombinantly produced or isolated RNA exerting a beneficial effect in the patient after cardiac arrest or any precursor or fragment thereof. In this aspect the administration of a recombinantly produced or isolated RNA increases the concentration of beneficial RNA in the subject to be treated. This higher concentration promotes the overall activity of the respective beneficial RNA in the subject. The fragments have to retain or essentially retain the function of the full-length RNA. Hence, the fragments have to be functional fragments. Such RNAs may also be expressed from a vector capable of producing such an RNA. Also orthologous or homologous sequences of the beneficial RNA from different species including precursors or functional fragments thereof may be used.

An RNA according to (ii) is in accordance with the present invention a RNA lowering or preventing the transcription of a maleficial RNAs. The compound inhibiting the expression of a maleficial RNA specifically inhibits the activity of said RNA. Preferably, the activity of a maleficial RNA is reduced by at least 50%, more preferred at least 75% such as at least 90% or 95%, even more preferred at least 98%, and most preferably about 100%. Means and methods for determining the reduction of activity of an RNA are established in the art and are described, for example, in Esau et al. (2004), JBC, 279:52361-52365 or Gribbings et al. (2009), Nature Cell Biology 11, 1143-1149.

An RNA according to (ii) is preferably a "siRNA". The term "siRNA" in accordance with the present invention refers to small interfering RNA, also known as short interfering RNA or silencing RNA. siRNAs are a class of 18 to 30, preferably 20 to 25, most preferred 21 to 23 or 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome. siRNAs have a well defined structure: a short double-strand of RNA (dsRNA), advantageously with at least one RNA strand having an overhang. Each strand typically has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Thus, any gene of which the sequence is known can in principle be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Also preferably at least one RNA strand has a 5'and/or 3'-overhang. Preferably, one or both ends of the double-strand have a 3'-overhang from 1-5 nucleotides, more preferably from 1-3 nucleotides and most preferably 2 nucleotides. In general, any RNA molecule suitable to act as siRNA is envisioned in the present invention. The most efficient silencing was so far obtained with siRNA duplexes composed of 21-nt sense and 21-nt antisense strands, paired in a manner to have 2-nt 3'overhangs. The sequence of the 2-nt 3' overhang makes a small contribution to the specificity of target recognition restricted to the unpaired nucleotide adjacent to the first base pair (Elbashir et al. Nature. 2001 May 24; 411(6836):494-8). 2'-deoxynucleotides in the 3' overhangs are as efficient as ribonucleotides, but are often cheaper to synthesize and probably more nuclease resistant.

An RNA according to (ii) is also preferably a "shRNA". A "shRNA" in accordance with the present invention is a short hairpin RNA, which is a sequence of RNA that makes a (tight) hairpin turn that can also be used to silence gene expression via RNA interference. shRNA preferably utilizes the U6 promoter for its expression. The shRNA hairpin structure is cleaved by the cellular machinery into siRNA, which is then bound to the RNA-induced silencing complex (RISC). This complex binds to and cleaves mRNAs which match the shRNA that is bound to it.

siRNAs and shRNAs are preferably chemically synthesized using appropriately protected ribonucleoside phosphoramidites and a conventional RNA synthesizer. Suppliers of RNA synthesis reagents include Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK).

Furthermore, an RNA according to (ii) is preferably an endoribonuclease-prepared siRNA (esiRNA). An esiRNA is a mixture of siRNA oligos resulting from cleavage of a long double-stranded RNA (dsRNA) according to item (b) with an endoribonuclease such as *Escherichia coli* RNase III or dicer. esiRNAs are an alternative concept to the usage of chemically synthesized siRNA for RNA Interference (RNAi). An esiRNAs is the enzymatic digestion of a long double stranded RNA in vitro. For the generation of esiRNAs a cDNA of an RNA template may be amplified by PCR and tagged with two bacteriophage-promotor sequences.

RNA polymerase is then used to generate long double stranded RNA that is complentary to the target-gene cDNA. This complentary RNA may be subsequently digested with RNase III from Escherichia coli to generate short overlapping fragments of siRNAs with a length between 18-25 base pairs. This complex mixture of short double stranded RNAs is similar to the mixture generated by Dicer cleavage in vivo and is therefore called endoribonuclease-prepared siRNA or short esiRNA. Hence, esiRNA are a heterogeneous mixture of siRNAs that all target the same mRNA sequence. esiRNAs lead to highly specific and effective gene silencing.

All these RNAs according to (i) and (ii) may be formulated, for example, as vesicles, such as liposomes. Liposomes have attracted great interest because of their specificity and the duration of action they offer from the standpoint of drug delivery. Liposomal delivery systems have been used to effectively deliver nucleic acids, such as siRNA in vivo into cells (Zimmermann et al. (2006) Nature, 441:111-114). Liposomes are unilamellar or multilamellar vesicles which have a membrane formed from a lipophilic material and an aqueous interior. The aqueous portion contains the composition to be delivered. Cationic liposomes possess the advantage of being able to fuse to the cell wall.

It also possible to promote the expression of a beneficial target RNA, and/or to inhibit the expression of a maleficial target RNA by using a CRISPR-Cas endonuclease-based construct (e.g. a CRISPR-Cas9-based construct, a CRISPR-Cpf1-based construct or a CRISPR- C2c1/2/3-based construct), a meganuclease, a zinc finger nuclease, or a transcription activator-like (TAL) effector (TALE) nuclease.

CRISPR/Cas endonuclease technologies are applicable in nearly all cells/model organisms and can be used for knocking out gene, chromosomal deletions, editing of DNA sequences and regulation (e.g. promoting) of gene expression. In particular, engineering of catalytically inactivated Cas variants (nuclease-deficient or nuclease-deactivated [dCas]) combined with transcriptional repressors, activators, or epigenetic modifiers enable sequence-specific regulation of gene expression and chromatin state (Lo and Qi (2017), F100Res. 2017 May 25;6. pii: F1000 Faculty Rev-747. doi: 10.12688/f1000research.11113.1). The regulation of the gene expression can be manipulated, for example, by the use of a catalytically dead Cas endonuclease enzyme (e.g. dCas9) that is conjugated with a transcriptional repressor to repress transcription a specific gene. In addition to coupling with transcriptional repressors, dCas9 can be fused with a transcriptional activator to drive target gene expression. This CRISPR-dCas9-based gene activation is termed CRISPR activation (CRISPRa). Similarly, catalytically inactive, "dead" Cpf1 nuclease (CRISPR from Prevotella and Francisella-1) can be fused to synthetic transcriptional repressors or activators to down- or up-regulate endogenous promoters controlling the target gene expression. Alternatively, engineered meganucleases, the DNA-binding domain of zinc finger nucleases (ZFNs) or transcription activator-like effector nucleases (TALENs) can be designed to specifically recognize the target gene or its promoter region or its 5'-UTR thereby, for example, inhibiting the expression of a target gene. Also the nuclease domains of engineered meganucleases, ZFNs or TALENs can be used to generate chimeric proteins which comprise besides the DNA-binding domain domains that activate or suppress gene expression.

The length of treatment needed to observe changes and the interval following treatment for responses to occur vary depending on the desired effect. The particular amounts may be determined by conventional tests which are well known to the person skilled in the art. Suitable tests are, for example, described in Tamhane and Logan (2002), "Multiple Test Procedures for Identifying the Minimum Effective and Maximum Safe Doses of a Drug", Journal of the American statistical association, 97(457):1-9.

The present description relates in a third aspect not part of the invention to a method for treating a patient after cardiac arrest, the method comprising a) predicting the neurological outcome of said patient according to the method of the first aspect of the invention; and b) providing cardiopulmonary support to the patient, performing organ transplantation in the patient, performing cardiac catheterization in the patient and/or implanting a pacemaker/an implanted cardioverter defibrillator (ICD) into the patient.

The above definitions and preferred embodiments of the method of the first and second aspect are equally applicable to the method of the third aspect.

As discussed herein above, patients with a predicted positive neurological outcome as determined by the methods of the first aspect of the invention have a high likelihood of survival and maintaining an independent life and may not require aggressive treatment for hypoxic brain damage. Accordingly, in particular such patients may be treated by more expensive or long-term oriented treatments that target the origin of the cardiac arrest. These include but are not limited to performing organ transplantation, performing cardiac catheterization and/or implanting a pacemaker/an implanted cardioverter defibrillator (ICD).

The present invention relates in a fourth aspect to the use of a binding agent which specifically binds to alpha-enolase in a method for predicting the neurological outcome of a patient after cardiac arrest.

The above definitions and preferred embodiments of the herein above described aspects of the invention are equally applicable to the use of the fourth aspect of the invention.

By "binding agent" is meant a molecule that has a binding affinity for another molecule. Binding agents include any substance capable of binding a component described herein, e.g. alpha-enolase.

Specific binding designates that the binding agent essentially does not or does not bind to other proteins or peptides than alpha-enolase. A binding agent is, for example, suitable for research purposes. For example, an antibody binding to the protein of the fourth aspect can be used in immuonassays, such as an ELISA or Western Blot. Immunoassays are biochemical tests that can measure the presence or concentration of alpha-enolase in a sample (e.g. a blood sample). This is equally applicable to a binding agent that specifically binds one of heat shock cognate 71 kDa protein, 4-3-3 protein zeta/delta, and cofilin-1.

By "binding affinity" is meant the strength of the total non-covalent interactions between a single binding site of a molecule (e.g., an antibody) and its binding partner (e.g., an antigen). Unless indicated otherwise, as used herein, "binding affinity" refers to intrinsic binding affinity, which reflects a specific interaction between members of a binding pair (e.g., antibody and antigen). The affinity of a molecule X for its partner Y can generally be represented by the dissociation constant ($K_d$). Affinity can be measured by standard methods known in the art, including those described herein. Low-affinity antibodies generally tend to dissociate readily, whereas high-affinity antibodies generally tend to remain bound longer.

The nature of the binding agent is not particularly limited as long as it essentially does not or does not bind to other proteins or peptides than alpha-enolase, heat shock cognate 71 kDa protein, 4-3-3 protein zeta/delta, or cofilin-1. Preferred examples will be provided herein below.

Also described herein is the use of a at least one binding agent which specifically binds to alpha-enolase, heat shock protein 71 kDa protein, 14-3-3 protein zeta/delta or cofilin-1 in a method for predicting the neurological outcome of a patient after cardiac arrest. The above definitions and preferred embodiments of the herein above described use of the fourth aspect of the invention are equally applicable to this use.

The present description relates in a fifth aspect not part of the invention to a kit for or predicting the neurological outcome of a patient after cardiac arrest, said kit comprising a binding agent which specifically binds to alpha-enolase.

The above definitions and preferred embodiments of the herein above described aspects of the invention are equally applicable to the kit of the fifth aspect.

The kits may be used in the methods of the present invention, in particular in a method for predicting the neurological outcome of a patient after having had a cardiac arrest as provided herein.

The kits (to be prepared in context) or the methods and uses of the invention may further comprise or be provided with (an) instruction manual(s). For example, said instruction manual(s) may guide the skilled person (how) to employ the kit in the methods and uses provided herein and in accordance with the present invention. Particularly, said instruction manual(s) may comprise guidance to use or apply the herein provided methods or uses.

The kit (to be prepared in context) may further comprise substances/chemicals and/or equipment suitable/required for carrying out the methods and uses of this invention. For example, such substances/chemicals and/or equipment are solvents, diluents and/or buffers for stabilizing and/or storing (a) compound(s) required for the uses provided herein, like stabilizing and/or storing the binding agent(s) comprised in the kits.

The kit may include a device having an immobilized binding agent on its surface in a pattern that generates a signal, wherein the immobilized binding agent is capable of specifically binding to alpha-enolase and optionally one or more further binding agents capable of specifically binding heat shock protein 71 kDa protein, 14-3-3 protein zeta/delta or cofilin-1. The kit may also include a detecting binding agent that specifically binds to at least one of the binding agents binding to alpha-enolase, heat shock protein 71 kDa protein, 14-3-3 protein zeta/delta or cofilin-1.

Also described herein is a kit for predicting the neurological outcome of a patient after cardiac arrest, said kit comprising at least one of the binding agents binding to alpha-enolase, heat shock protein 71 kDa protein, 14-3-3 protein zeta/delta or cofilin-1. The above definitions and preferred embodiments of the herein above described kit of the fifth aspect are equally applicable to this kit

In accordance with a preferred embodiment of the fourth and fifth aspect the use or kit additionally comprises one or more agents specifically binding to heat shock cognate 71 kDa protein, 14-3-3 protein zeta/delta or cofilin-1.

Hence, also in connection with this use in addition to a binding agent which specifically binds to alpha-enolase preferably one or more binding agents which specifically bind heat shock cognate 71 kDa protein, a binding agent which is/are used. In this connection "one or more" is preferably two or three and most preferably three.

Similarly, the kit in addition to a binding agent which specifically binds to alpha-enolase preferably comprises one or more of binding agents which specifically binds heat shock cognate 71 kDa protein, a binding agent which specifically binds 4-3-3 protein zeta/delta, and a binding agent which specifically binds cofilin-1 may be used. In this connection "one or more" is preferably two or three and most preferably three.

The above statements on a binding agent which specifically binds to alpha-enolase apply *mutatis mutandis* to agents specifically binding to heat shock cognate 71 kDa protein, 14-3-3 protein zeta/delta or cofilin-1. The additional use of one or more of these binding agents further increases the liability of the prediction of the neurological outcome of a patient after cardiac arrest.

In accordance with a preferred embodiment of the fourth and fifth aspect, the binding agent(s) is/are selected from antibodies, antibody mimetics, small molecules and aptamers.

The term "antibody" as used in accordance with the present invention comprises, for example, polyclonal or monoclonal antibodies. Furthermore, also derivatives or fragments thereof, which still retain the binding specificity to the target, e.g. alpha-enolase, are comprised in the term "antibody". Antibody fragments or derivatives comprise, inter alia, Fab or Fab' fragments, Fd, F(ab')2, Fv or scFv fragments, single domain VH or V-like domains, such as VhH or V-NAR-domains, as well as multimeric formats such as minibodies, diabodies, tribodies or triplebodies, tetrabodies or chemically conjugated Fab'-multimers (see, for example, Harlow and Lane "Antibodies, A Laboratory Manual", Cold Spring Harbor Laboratory Press, 198; Harlow and Lane "Using Antibodies: A Laboratory Manual" Cold Spring Harbor Laboratory Press, 1999; Altshuler EP, Serebryanaya DV, Katrukha AG. 2010, Biochemistry (Mosc)., vol. 75(13), 1584; Holliger P, Hudson PJ. 2005, Nat Biotechnol., vol. 23(9), 1126). The multimeric formats in particular comprise bispecific antibodies that can simultaneously bind to two different types of antigen. The first antigen can be found on the protein of the invention. The second antigen may, for example, be a tumor marker that is specifically expressed on cancer cells or a certain type of cancer cells. Non-limting examples of bispecific antibodies formats are Biclonics (bispecific, full length human IgG antibodies), DART (Dual-affinity Re-targeting Antibody) and BiTE (consisting of two single-chain variable fragments (scFvs) of different antibodies) molecules (Kontermann and Brinkmann (2015), Drug Discovery Today, 20(7):838-847).

The term "antibody" also includes embodiments such as chimeric (human constant domain, non-human variable domain), single chain and humanised (human antibody with the exception of non-human CDRs) antibodies.

Various techniques for the production of antibodies are well known in the art and described, e.g. in Harlow and Lane (1988) and (1999) and Altshuler et al., 2010, loc. cit. Thus, polyclonal antibodies can be obtained from the blood of an animal following immunisation with an antigen in mixture with additives and adjuvants and monoclonal antibodies can be produced by any technique which provides antibodies produced by continuous cell line cultures. Examples for such techniques are described, e.g. in Harlow E and Lane D, Cold Spring Harbor Laboratory Press, 1988; Harlow E and Lane D, Using Antibodies: A Laboratory Manual, Cold Spring Harbor Laboratory Press, 1999 and include the hybridoma technique originally described by Köhler and Milstein, 1975, the trioma technique, the human B-cell hybridoma technique (see e.g. Kozbor D, 1983, Immunology Today, vol.4, 7; Li J, et al. 2006, PNAS, vol. 103(10), 3557) and the EBV-hybridoma technique to produce human monoclonal antibodies (Cole et al., 1985, Alan R. Liss, Inc, 77-96). Furthermore, recombinant antibodies may be obtained from monoclonal antibodies or can be prepared de novo using various display methods such as phage, ribosomal, mRNA, or cell display. A suitable system for the expression of the recombinant (humanised) antibodies may be selected from, for example, bacteria, yeast, insects, mammalian cell lines or transgenic animals or plants (see, e.g., US patent 6,080,560; Holliger P, Hudson PJ. 2005, Nat Biotechnol., vol. 23(9), 11265). Further, techniques described for the production of single chain antibodies (see, inter alia, US Patent 4,946,778) can be adapted to produce single chain antibodies specific for an epitope, such as an epitope of alpha-enolase. Surface plasmon resonance as employed in the BIAcore system can be used to increase the efficiency of phage antibodies.

As used herein, the term "antibody mimetics" refers to compounds which, like antibodies, can specifically bind antigens, such as the alpha-enolase in the present case, but which are not structurally related to antibodies. Antibody mimetics are usually artificial peptides or proteins with a molar mass of about 3 to 20 kDa. For example, an antibody mimetic may be selected from the group consisting of affibodies, adnectins, anticalins, DARPins, avimers, nanofitins, affilins, Kunitz domain peptides and Fynomers®. These polypeptides are well known in the art and are described in further detail herein below.

The term "affibody", as used herein, refers to a family of antibody mimetics which is derived from the Z-domain of staphylococcal protein A. Structurally, affibody molecules are based on a three-helix bundle domain which can also be incorporated into fusion proteins. In itself, an affibody has a molecular mass of around 6kDa and is stable at high temperatures and under acidic or alkaline conditions. Target specificity is obtained by randomisation of 13 amino acids located in two alpha-helices involved in the binding activity of the parent protein domain (Feldwisch J, Tolmachev V.; (2012) Methods Mol Biol. 899:103-26).

The term "adnectin" (also referred to as "monobody"), as used herein, relates to a molecule based on the 10th extracellular domain of human fibronectin III (10Fn3), which adopts an Ig-like β-sandwich fold of 94 residues with 2 to 3 exposed loops, but lacks the central disulphide bridge (Gebauer and Skerra (2009) Curr Opinion in Chemical Biology 13:245-255). Adnectins with the desired target specificity, e.g. against alpha-enolase, can be genetically engineered by introducing modifications in specific loops of the protein.

The term "anticalin", as used herein, refers to an engineered protein derived from a lipocalin (Beste G, Schmidt FS, Stibora T, Skerra A. (1999) Proc Natl Acad Sci USA. 96(5):1898-903; Gebauer and Skerra (2009) Curr Opinion in Chemical Biology 13:245-255). Anticalins possess an eight-stranded D-barrel which forms a highly conserved core unit among the lipocalins and naturally forms binding sites for ligands by means of four structurally variable loops at the open end. Anticalins, although not homologous to the IgG superfamily, show features that so far have been considered typical for the binding sites of antibodies: (i) high structural plasticity as a consequence of sequence variation and (ii) elevated conformational flexibility, allowing induced fit to targets with differing shape.

As used herein, the term "DARPin" refers to a designed ankyrin repeat domain (166 residues), which provides a rigid interface arising from typically three repeated β-turns. DARPins usually carry three repeats corresponding to an artificial consensus sequence, wherein six positions per repeat are randomised. Consequently, DARPins lack structural flexibility (Gebauer and Skerra, 2009).

The term "avimer", as used herein, refers to a class of antibody mimetics which consist of two or more peptide sequences of 30 to 35 amino acids each, which are derived from A-domains of various membrane receptors and which are connected by linker peptides. Binding of target molecules occurs via the A-domain and domains with the desired binding specificity, e.g. for alpha-enolase, can be selected, for example, by phage display techniques. The binding specificity of the different A-domains contained in an avimer may, but does not have to be identical (Weidle UH, et al., (2013), Cancer Genomics Proteomics; 10(4):155-68).

A "nanofitin" (also known as affitin) is an antibody mimetic protein that is derived from the DNA binding protein Sac7d of Sulfolobus acidocaldarius. Nanofitins usually have a molecular weight of around 7kDa and are designed to specifically bind a target molecule, such as e.g. alpha-enolase, by randomising the amino acids on the binding surface (Mouratou B, Béhar G, Paillard-Laurance L, Colinet S, Pecorari F., (2012) Methods Mol Biol.; 805:315-31).

The term "affilin", as used herein, refers to antibody mimetics that are developed by using either gamma-B crystalline or ubiquitin as a scaffold and modifying amino-acids on the surface of these proteins by random mutagenesis. Selection of affilins with the desired target specificity, e.g. against alpha-enolase, is effected, for example, by phage display or ribosome display techniques. Depending on the scaffold, affilins have a molecular weight of approximately 10 or 20kDa. As used herein, the term affilin also refers to di- or multimerised forms of affilins (Weidle UH, et al., (2013), Cancer Genomics Proteomics; 10(4):155-68).

A "Kunitz domain peptide" is derived from the Kunitz domain of a Kunitz-type protease inhibitor such as bovine pancreatic trypsin inhibitor (BPTI), amyloid precursor protein (APP) or tissue factor pathway inhibitor (TFPI). Kunitz domains have a molecular weight of approximately 6kDA and domains with the required target specificity, e.g. against alpha-enolase, can be selected by display techniques such as phage display (Weidle et al., (2013), Cancer Genomics Proteomics; 10(4):155-68).

As used herein, the term "Fynomer®" refers to a non-immunoglobulin-derived binding polypeptide derived from the human Fyn SH3 domain. Fyn SH3-derived polypeptides are well-known in the art and have been described e.g. in Grabulovski et al. (2007) JBC, 282, p. 3196-3204, WO 2008/022759, Bertschinger et al (2007) Protein Eng Des Sel 20(2):57-68, Gebauer and Skerra (2009) Curr Opinion in Chemical Biology 13:245-255, or Schlatter et al. (2012), MAbs 4:4, 1-12).

The "small molecule" as used herein is preferably an organic molecule. Organic molecules relate or belong to the class of chemical compounds having a carbon basis, the carbon atoms linked together by carbon-carbon bonds. The original definition of the term organic related to the source of chemical compounds, with organic compounds being those carboncontaining compounds obtained from plant or animal or microbial sources, whereas inorganic compounds were obtained from mineral sources. Organic compounds can be natural or synthetic. The organic molecule is preferably an aromatic molecule and more preferably a heteroaromatic molecule. In organic chemistry, the term aromaticity is used to describe a cyclic (ring-shaped), planar (flat) molecule with a ring of resonance bonds that exhibits more stability than other geometric or connective arrangements with the same set of atoms. Aromatic molecules are very stable, and do not break apart easily to react with other substances. In a heteroaromatic molecule at least one of the atoms in the aromatic ring is an atom other than carbon, e.g. N, S, or O. For all above-described organic molecules the molecular weight is preferably in the range of 200 Da to 1500 Da and more preferably in the range of 300 Da to 1000 Da.

Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 Da, or less than about 1000 Da such as less than about 500 Da, and even more preferably less than about Da amu. The size of a small molecule can be determined by methods well-known in the art, e.g., mass spectrometry. The small molecules may be designed, for example, based on the crystal structure of the target molecule, where sites presumably responsible for the biological activity can be identified and verified in *in vivo* assays such as in vivo high-throughput screening (HTS) assays.

Aptamers are nucleic acid molecules or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

Nucleic acid aptamers are nucleic acid species that normally consist of (usually short) strands of oligonucleotides. Typically, they have been engineered through repeated rounds of in vitro selection or equivalently, SELEX (systematic evolution of ligands by exponential enrichment) to bind to various molecular targets such as small molecules, proteins, nucleic acids, and even cells, tissues and organisms.

Peptide aptamers are usually peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein, the variable peptide loop being inserted within the redoxactive site, which is a -Cys-Gly-Pro-Cys-loop (SEQ ID NO: 55) in the wild protein, the two cysteins lateral chains being able to form a disulfide bridge. Peptide aptamer selection can be made using different systems, but the most widely used is currently the yeast two-hybrid system.

Aptamers offer the utility for biotechnological and therapeutic applications as they offer molecular recognition properties that rival those of the commonly used biomolecules, in particular antibodies. In addition to their discriminatory recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamers' inherently low molecular weight. Unmodified aptamer applications currently focus on treating transient conditions such as blood clotting, or treating organs such as the eye where local delivery is possible. This rapid clearance can be an advantage in applications such as in vivo diagnostic imaging. Several modifications, such as 2'-fluorine-substituted pyrimidines, polyethylene glycol (PEG) linkage, fusion to albumin or other half life extending proteins etc. are available to scientists such that the half-life of aptamers can be increased for several days or even weeks.

Unless otherwise defined, all technical and scientific terms used herein have the same meaning as commonly understood by one of ordinary skill in the art to which this invention belongs. In case of conflict, the patent specification, including definitions, will prevail.

Regarding the embodiments characterised in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

The methods and techniques of the present invention are generally performed according to conventional methods well known in the art and as described in various general and more specific references that are cited and discussed throughout the present specification unless otherwise indicated. See, e.g., Sambrook et al., Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual, Cold Spring Harbor Laboratory Press, Cold Spring Harbor, N.Y. (1990).

While the invention is illustrated and described in detail in the drawings and foregoing description, such illustration and description are to be considered illustrative or exemplary and not restrictive.

The invention also covers all further features shown in the figures individually, although they may not have been described in the previous or following description. Also, single alternatives of the embodiments described in the figures and the description and single alternatives of features thereof can be disclaimed from the subject matter of the other aspect of the invention.

Furthermore, in the claims the word "comprising" does not exclude other elements or steps, and the indefinite article "a" or "an" does not exclude a plurality. A single unit may fulfil the functions of several features recited in the claims. The terms "essentially", "about", "approximately" and the like in connection with an attribute or a value particularly also define exactly the attribute or exactly the value, respectively. Any reference signs in the claims should not be construed as limiting the scope.

The Figures show.

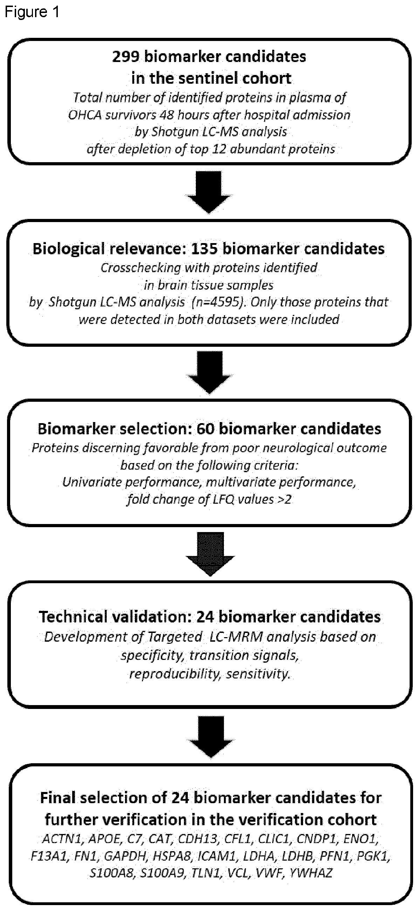

Figure 1. Schematic flow chart of experimental proteomic approach for biomarker discovery. Abbreviations: LC-MS - liquid chromatography-mass spectrometry; LFQ - label-free quantification; MRM - multiple reaction monitoring; Gene names were used for protein nomenclature.

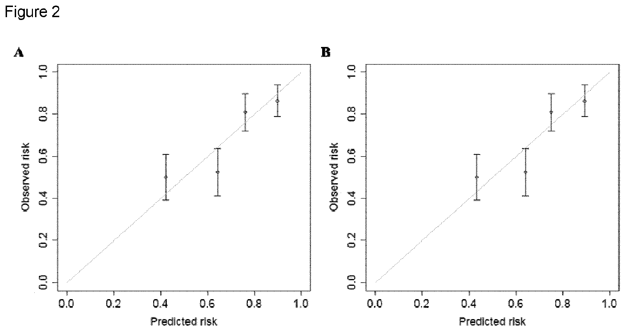

Figure 2. Calibration plots of model M3 and M4 are shown in panel A and panel B, respectively. These calibration plots evaluate the agreement of predicted and observed risk for poor neurological outcome. The risk comparison is grouped by quartiles of the predicted risk estimated by M3 and M4. 95% confidence intervals are added for the observed risk. Optimal calibration is revealed if the mean predicted and observed risk in each of the four groups agree, i.e., if all dots lie on the diagonal grey line. Here, the 95% confidence intervals intersect the diagonal which confirms a reasonable calibration of M3 and M4.

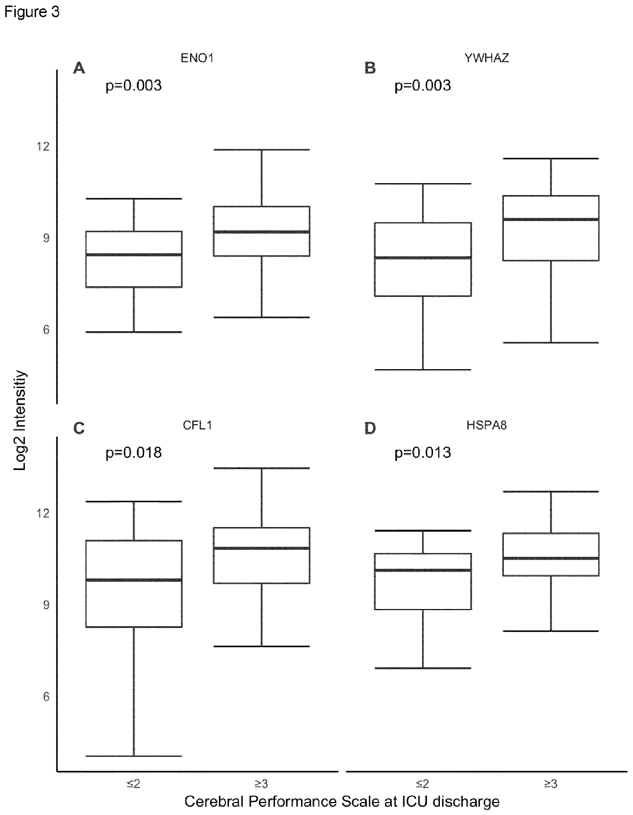

**Figure 3.** Comparison of plasma abundance levels indicated by log2 intensities of alpha-enolase (A), 14-3-3 protein zeta/delta (B), cofilin-1 (C) and HSPA8 (D) between patients with good neurological outcome (Cerebral Performance Categories (CPC) $\leq$ 2) and negative neurological outcome (CPC$\geq$3).

The examples illustrate the invention.

## Examples

In the prospective, observational, single-center study described herein below all out-of-hospital cardiac arrest (OHCA) survivors admitted to the emergency department of a university-affiliated tertiary care centre were enrolled and neurological outcome was assessed by the Cerebral Performance Categories (CPC) score at discharge. To identify biomarkers for negative neurological outcome (CPC score $\geq$ 3) in plasma samples collected 48 hours after OHCA, a three-step proteomics strategy of preselection by shotgun analyses, crosschecking in brain tissue samples and verification by targeted proteomic analyses in combination with a discovery and validation statistical approach was performed.
96 OHCA survivors were included of whom 63 patients (66%) had a negative neurological outcome. Out of a total of 299 plasma proteins, identified alpha-enolase, 14-3-3 protein zeta/delta, cofilin-1 and Heat shock cognate 71 kDa protein were identified as novel biomarkers for negative neurological outcome. The implementation of these proteomically identified biomarkers into a clinical multimarker model resulted in a significant improvement of neurological outcome prediction (C-index 0.70) compared to traditional risk factors alone (C-index 0.67; p=0.019). Thus, explained variation of the neurological outcome could be significantly improved from 10.0% to 11.9% (Table 10, in particular Model 2 vs. Model 4).

## Example 1 - Experimental outline and methods

### Study design and patients

The study was designed as a combination of a pilot basic science study and a clinical verification trial: a sentinel cohort was used to identify potential plasma biomarker candidates for negative neurological outcome by comparing the proteomic profile between patients with positive and negative neurological outcome. The clinical verification study was designed to establish biomarker candidates in a larger study population (verification cohort).
OHCA patients were consecutively recruited on arrival at the emergency department of the Medical University of Vienna, a university-affiliated tertiary care center, and were followed up to intensive care unit (ICU) discharge. Adult patients suffering non-traumatic, normothermic OHCA due to cardiac disorders, respiratory failure, or hemodynamic or metabolic factors with a Glasgow Coma Scale of three at admission were eligible. Patients with history of previous cardiac arrest, coexisting neurological disorders or neoplasms of the central nervous system, psychiatric illness, alcohol or drug misuse and those with ongoing psychotropic medication as identified by the attending or ward physician were excluded. Moreover, patients with abnormal findings in the cranial computed tomography, including hydrocephalus and shunt artifacts, severe movement artifacts, intracerebral hemorrhage or chronic large ischemic lesions were excluded. The sentinel cohort was composed by a random selection of five patients with positive and five patients with negative neurological outcome using a pseudorandom number generator (Urbaniak et al. (2013) Research Randomizer, Version 4.0. The remaining study participants were assigned to the verification cohort.

### Outcomes

The primary study endpoint was the neurological outcome at discharge from the ICU assessed by an experienced neurologist using the Cerebral Performance Categories (CPC) score, which measures outcome on a five-point scale (1=good cerebral performance, 2=moderate cerebral disability, 3=severe cerebral disability, 4=coma or vegetative state, and 5= brain death). Neurological outcome was dichotomized into positive neurological outcome (CPC $\leq$2) and negative neurological outcome (CPC score $\geq$ 3).

### Study procedures

Cardiopulmonary resuscitation (CPR) and post-resuscitation management were performed according to current guidelines (Callaway et al. (2015) Circulation 132 (16 Suppl. 1):S84-145. All patients were routinely treated with targeted temperature management for 24 hours. Data on cardiac arrest for individual patients were recorded according to 'Utstein Style Criteria' (Jacobs et al. (2004) Circulation 110(21):3385-97).

Blood samples were drawn and immediately placed on ice from all patients 48 hours after hospital admission. Neuron-specific enolase (NSE) and protein S100-B (S100B) levels were determined by electrochemiluminescence immunoassays according to the local laboratory's standard procedure. Additional plasma samples were stored at -80°C and applied for proteomic analyses immediately after thawing. Brain tissue especially vulnerable to hypoxia (striatum, hippocampus and periventricular white matter) was harvested postmortem from patients without significant neurological symptoms or substantial neuropathological alterations who died for other causes than OHCA by the Institute of Neurology, Medical University of Vienna and processed for proteomic analyses.

The experimental procedure is detailed further below. Depletion of top twelve abundant proteins and tryptic in-solution digestion were applied as preparation steps to plasma samples. After sonication of brain tissue samples in sample buffer, soluble proteins were digested.

The shotgun proteomics analysis was conducted on a Q-Exactive Orbitrap (Thermo Fisher Scientific, USA) coupled with an UltiMate 3000 RSLC nano System (Dionex, USA) (Muqaku et al. (2017) Proteomics 16(1):86-99). For the evaluation of shotgun liquid chromatography-mass spectrometry (LC-MS) data, the MaxQuant 1.5.2.8 software tools were used (Cox et al. (2008) Nature biotechnology 26(12):1367-72).

Targeted analysis was performed on an Agilent 6490 triple quadrupole mass spectrometer coupled with a nano-Chip-LC Agilent Infinity Series HPLC1260 system (Agilent Technologies, USA). Skyline software was used for MRM method development, as described recently (Muqaku, as above). Protein abundance levels were indicated as log2 intensities.

**Statistical analysis**

*Pre-selection of biomarker candidates:* Quantification and statistical analyses of shotgun LC-MS data were based on label-free quantification (LFQ) values using Perseus software as previous described (Muqaku, as above). Proteins with significantly different LFQ values and none significantly regulated proteins with a fold change of LFQ values higher than two between patients with negative and positive neurological outcome were taken into consideration for MRM assay development. Detailed information are provided herein below. Each of the biomarker candidates were tested for their ability to predict neurological outcome using univariate regression analyses. Wilcoxon signed-rank test was used to compare protein abundance levels between patients with negative and positive neurological outcome.

*Development and evaluation of a proteomic signature:* The pre-selected biomarker set was further considered to identify a proteomic signature discriminating patients with positive neurological outcome from those with negative outcome with the verification cohort of n=86. This sample size was based on feasibility and the sample size of other prospective studies performed on a similar patient population (Cronberg et al. (2011) Neurology 77(7):623-30). Any more formal sample size calculation for this biomarker finding study would have required strong and a-priorily unestimable assumptions on the number, the expression and the correlation of the proteomic biomarkers, and their combined effect on the outcome. Some variables were transformed due to high frequency of zeros and high skewness, and missing values were imputed using chained equations.

Three blocks of variables available at day two after ROSC were considered for model building. The first block consisted of well-established predictors of neurological outcomes such as age, shockable rhythm, time from cardiac arrest to start of life support, time from CPR to ROSC, pH, serum lactate in mmol/L, and hemoglobin in g/dl. As a second block, adrenalin was added in mg administrated during resuscitation, witnessed cardiac arrest, sex, NSE, and S100B. The third block consisted of 24 proteomic features. A ridge logistic regression model (M1) was fitted with the first block of variables, and linear predictors from that model were computed (Hoerl et al. (1970) Technometrics 12(1):55-67). The linear predictors from M1 were used as offsets in a LASSO logistic regression model (M2), where the second block of potential predictors was evaluated. The third model (M3) extended M2 by proteomic markers in the third block. In M2 and M3, all variables of the second and third blocks were competing for selection. The penalty parameter was optimized by evaluating the 10-fold cross-validated deviance (CVD). A fourth model (M4) was obtained by re-fitting M3 with elastic-net logistic regression as an alternative selection method (Zou et al. (2005) Journal of the Royal Statistical Society Series B 67(2):301-20).

To evaluate the model building strategy, 10-fold cross-validation was used and the concordance index (area under the ROC curve), the explained variation expressed as discrimination slope, and the calibration slope was computed. Calibration was graphically evaluated. Model stability was investigated by computing bootstrap inclusion frequencies for each variable. A non-parametric bootstrap test to evaluate the added value of blocks of variables was used.

Descriptive analyses comprised computation of medians and interquartile ranges for continuous variables, and absolute and relative frequencies for categorical variables. More details on statistical model building are provided further below.

*Baseline characteristics:* One-hundred OHCA patients were included with a median age of 58 years (IQR, 49-69). Four patients were excluded because of poor specimen quality. Hence, the final study population consisted of 96 patients, of whom 63 patients (66 percent) had a negative neurological outcome at discharge from the ICU. Detailed baseline characteristics are displayed in Table 1.

*Identification of biomarker candidates:* The flow chart of proteomic analyses is illustrated in Figure 1. A total of 299 proteins were identified in plasma samples from the sentinel population by proteomic shotgun analyses. After crosschecking with the proteome of 10 independent brain autopsy samples (4595 proteins), 135 proteins were detected within plasma as well as brain tissue samples. Of these proteins, 60 proteins fulfilled selection criteria and were taken into consideration for MRM assay development. Finally an MRM method was established as potential prognostic signature, consisting of 24 proteins represented by 40 proteotypic peptides and 100 optimized mass transitions in the triplequadrupol MS that was applied for measurement in the verification cohort (Tables 2 and 3). Protein abundance values of MRM analyses are summarized in Table 4.

*Prognostic model:* The results of a univariate regression analysis of each of the 24 biomarker candidates to predict negative neurological outcome are outlined in Table 5. To explore the best multivariable combination of clinical parameters and biomarkers to predict neurological outcome in OHCA patients, a multi-stage statistical approach was applied. Model M1, including all well-established neurological outcome predictors, achieved a discriminative ability (cross-validated c-index) of 0.67 (Table 6). This c-index increased to 0.68 by the variable selection process of model M2, which added S100-B (Table 7). Model M3 additionally selected a multivariable combination of the proteomically measured biomarker candidates (n=24) and yielded a c-index of 0.70 (Table 8). This optimal combination, consisting of alpha-enolase, 14-3-3 protein zeta/delta and cofilin-1, resulted in a significant improvement in neurological outcome prediction compared to M2 (p=0.025 for added value). M4, which employed elastic-net as a less restrictive variable selection procedure, additionally selected Heat shock cognate 71 kDa protein (HSPA8), yielding similar performance (c-index= 0.70; p= 0.019 for added value compared to M2; Table 9). Explained variation of the outcome could be improved from 8.2% in M1 to 10.0% in M2 and to 12.2% in M3 and to 11.9% in M4 (Table 10). Figure 2 depicts the agreement between predicted and observed risk for negative outcome and confirms a reasonable calibration of M3.

As illustrated in Figure 3, plasma levels of alpha-enolase (9.18, IQR 8.39-10.02 versus 8.44, IQR 7.37-9.20; P=0.003), 14-3-3 protein zeta/delta (9.59, IQR 8.24-10.36 versus 8.34, IQR 7.08-9.48; P=0.003), cofilin-1 (10.85, IQR 9.69-11.52 versus 9.80, IQR 8.27-11.10; P=0.018) and HSPA8 (10.51, IQR 9.94-11.33 versus 10.12, IQR 8.84-10.67; P=0.013) were significantly elevated in patients with negative neurological outcome compared to patients with positive outcome. Table 11 summarizes the calculated percentage changes relative to the median levels of each biomarker that allow prediction of a positive or negative neurological outcome. The values were calculated to have high specificity, so that no patient with a positive neurological outcome is falsely predicted to have a negative neurological outcome.

## Experimental methods

*Plasma depletion:* For proteomic analyses, plasma samples were depleted employing Pierce™ Top 12 Abundant Protein Depletion Spin Columns (Thermo Fisher Scientific, USA). The depletion was performed according to the protocol of the manufacturer, using 14 μL of plasma for shotgun analysis and 550 μg total protein amount of plasma per depletion for targeted analysis. Protein digestion was performed using 257 μl of depleted plasma containing not more than 25 μg total protein amount for shotgun analysis and for targeted analysis 20 μg total protein amount was used. Bradford assay was applied to all samples for determination of total protein concentration before and after depletion.

*Preparation of brain tissue samples*: Brain tissue samples were sonicated for several seconds using a ultra-sonicator after adding of 100μl sample puffer. The supernatant containing soluble proteins was collected after centrifugation of sonicated sample for 10 min at 14000 g. For the protein digestion 20 μg total protein amount were used. Sample buffer compositions: 45% urea (Merck, Germany), 11.4% thiourea (Sigma-Aldrich, USA), 4% CHAPS (Gerbu, Germany), 0.05% sodium dodecyl sulfate (Gerbu) and 0.1M dithiothreitol (Gerbu).

*In-solution digestion:* In-solution digestion was performed using 10 kDa MW cut off filters (Nanosep with Omega membrane, USA), which consist on protein reduction with 32mM dithiothreitol (Gerbu) and alkylation of reduced proteins with 54 mM 2-iodoacetamide solution (Sigma Aldrich). Both, dithiothreitol and 2-iodoacetamide solutions were prepared with 8 M guanidinium hydrochloride (Sigma Aldrich) solution in 50 nM ammonium bicarbonate. Afterwards, proteins were digested on the filter using a Trypsin/Lys-C Mix (MS grade; Promega, USA) with a total enzyme to protein ratio of 1:20. For clean-up of resulting peptide samples C18 spin columns (Pierce™ C18 Spin Columns, Thermo Fisher Scientific, USA) were used and, after drying via vacuum centrifugation, the samples were stored at -20°C. Upon liquid chromatography-mass spectrometry (LC-MS) analysis the dried peptides were reconstituted in 5 μL of the equimolar 10 fmol standard peptide mix and 40 μL of mobile phase A (98% H2O, 2% ACN, 0.1% FA) for shotgun analysis, and for targeted analysis in 30 μL of the 10 fmol/μL standard peptide mix solution containing 30% formic acid. Standard peptides [M28 - TTPA VLDSDGSYFLYSK; HK0 - VLETKSLYVR; HK1 - VLETK(ε-AC)SLYVR] were obtained from Sigma and Peptide Specialty Laboratories GmbH and spiked in each sample as internal quality control for monitoring LC-MS-system stability.

*Shotgun LC-MS analysis:* The shotgun analysis of plasma as well as brain tissue samples were conducted on the same mass spectrometric equipment by applying the same setting for protein identification and MS1 based protein quantification as published recently. Briefly, peptides were separated by UltiMate 3000 RSLC nano System (Pre-column: Acclaim PepMap 100, C18 100 $\mu$m x 2 cm; Analytical column: Acclaim PepMap RSLC C18 75 $\mu$m $\times$ 50cm; Dionex, USA). Peptides of plasma samples were eluted to the analytical column by applying a gradient from 8 to 40% mobile phase B (80% ACN, 2% H2O, 0.1% FA) over 43 min and operating at a flow rate of 300nL/min. Whereas peptides generated from digestion of brain tissue samples were separated by applying a gradient from 8% to 40% over 235 min.

Data acquisition was conducted on a QExactive mass spectrometer (Thermo Fischer Scientific, USA) using a top 8 data dependent method. Protein identification was achieved using the MaxQuant 1.5.2.8 software and searching against the UniProt database (version 102014 with 20 195 entries). Search criteria included a maximum of two missed cleavages. Additionally, a minimum of two peptide identifications per protein (including one unique) was requested and an false discovery rate of less than 0.01 was applied at both peptide and protein level. Carbamidomethylation of cysteines was set as fixed modification and methionine oxidation as well as N-terminal protein acetylation as variable modifications.

Shotgun analyses were performed in plasma samples of OHCA patients with negative and positive neurological outcome. A MS1-based label-free quantification approach and statistical analysis was applied to quantify the identified proteins based on label free quantification (LFQ) values using Perseus. Up- and downregulated proteins were determined applying a two-sided t test. The significance threshold of p-value < 0.05 (minimum fold change of two of LFQ values) was calculated using permutation-based multiparameter correction. Only proteins fulfilling the following selection criteria were taken into consideration for multiple reaction monitoring (MRM) assay development: multi-parameter significantly regulated proteins; none significantly regulated proteins with p-value lower than 0.05 and none significantly regulated proteins with fold change of LFQ values (up- or down regulated) higher than 2. In order to identify brain-derived plasma proteins, these biomarker candidates were crosschecked with the proteome of brain autopsy samples.

*Targeted LC-MRM analysis:* Targeted analysis was performed on an Agilent 6490 triple quadrupole mass spectrometer coupled with a nano-Chip-LC Agilent Infinity Series HPLC1260 system, as described recently. Solvent compositions were 97.8% H2O, 2% ACN and 0.2% FA for solvent A, and 97.8% ACN, 2% H2O and 0.2% FA for solvent B.

*MRM Assay Development:* The MRM method was developed based on high resolution orbitrap shotgun data and using same plasma sample measured with shotgun approach. Applied criteria for the development of MRM method were recently published. Briefly, only peptides with unique amino acid sequence and peptides without missed cleavage were considered for further optimization. Additionally, peptides containing methionine residue were excluded from the list of targeted peptides. Best transitions and interference-free transitions were selected by applying of a unscheduled MRM method in multiple sample injections. In the final step 86 plasma sample were measured in technical duplicates with developed scheduled MRM method by using 3.5 minutes time window and cycle time of 1300 milliseconds. The MRM method development was implemented using Skyline software (v. 3.1), allowing data analysis and data preparation for statistical analysis. The manual inspection regarding correct peak selection, interferences, and integration boundaries as well as normalization to standard peptides of the data was done with Skyline. Additionally, the data were normalized to the sample volume used for depletion and digestion. In the next step, data were multiplied with $10^9$ and were log2 transformed. Finally, the abundance at protein level was calculated as mean value over two technical duplicates of all transition signals generated from all peptides belonging to a protein.

## Statistical methods

*Variable transformation and imputation of missing values for statistical model building:* For statistical model building, some variables were transformed due to high frequency of zeros and high skewness. All biomarker were log2-transformed. Time from CPR to the return of spontaneous circulation (ROSC) in minutes was transformed to log2(x+1). Time from cardiac arrest to start of life support in minutes was dichotomized into 'immediate' and 'not immediate'. The pH level was measured as the maximum absolute difference of pH to 7.3pH within the first 24 hours. For serum lactate, the maximum value measured within the first 24 hours was used in the analysis. Missing values in 6 clinical variables (max 23%) were imputed using chained equations.

*Validation of model building strategy and model stability*: An outer 10-fold cross-validation loop was used to evaluate the model building strategy. In particular, the concordance index (area under the ROC curve) was computed, the explained variation expressed as discrimination slope and the calibration slope. Calibration was also graphically evaluated by calibration plots, showing the observed vs. the predicted rates of negative neurological outcome. Model stability was investigated by computing bootstrap inclusion frequencies for each variable, if the model building process was repeated in 1000 bootstrap resamples drawn with replacement from the original data set.

*Test for added value of blocks of variables:* M1 was tested against a null model by randomly permuting the outcome status of the patients 1000 times, refitting M1 to each of the permuted data sets and computing a p-value as the proportion of permuted CVD values which were less than or equal to CVD of M1 in the original data set. M2 was tested against M1 as follows. First, M1 and M2 were refitted in 1000 resamples drawn using replacement from the original data. The resulting two linear predictors $X\hat{\beta}_1^b$ and $X\hat{\beta}_2^b$ obtained in each of the bootstrap samples b (b=1,...,1000) were then computed for each observation of the original data set, and another bivariable regression model was fitted to the original data set including $X\hat{\beta}_1^b$ and $X\hat{\beta}_2^b$ as the only predictors, estimating coefficients $\hat{\gamma}_1^b$ and $\hat{\gamma}_2^b$. Finally, a two-sided p-value for testing the null hypothesis of no added value of M2 on top of M1 was obtained by applying the 'twice smaller tail' rule to the empirical distribution of the 1000 values of $\hat{\gamma}_2^b$. In particular, the *p*-value was calculated as

$$p = 2\min\left\{\frac{1}{1000}\sum_{b=1}^{1000} I\{\hat{\beta}_{LPblock2}^b < 0\}, \frac{1}{1000}\sum_{b=1}^{1000} I\{\hat{\beta}_{LPblock2}^b > 0\}\right\}$$
$$+ \frac{1}{1000}\sum_{b=1}^{1000} I\{\hat{\beta}_{LPblock2}^b = 0\}$$

where $I\{\cdot\}$ = 1 if condition $\{\cdot\}$ is true and 0 otherwise. Similarly, M3 and M4 were tested against M2. This procedure modifies the one proposed by de Bin et al. to be applicable in internal validation. The non-parametric bootstrap test was described by Efron and Tibshirani. R Version 3.3.2 (R development core team, Vienna, 2017) and the glmnet R package for fitting the ridge was used.

Table 1. Patient baseline characteristics of total study population (n=96).

| | Total study population (n=96) |
|---|---|
| Age, yr, median (IQR) | 58 (49-69) |
| Female sex (%) | 22 (22.9) |
| Cardiac arrest witnessed | 79 (82.3) |
| **Reason for cardiac arrest (%)** | |
| Cardiac, n (%) | 83 (86.5) |
| Pulmonal, n (%) | 11 (11.5) |
| Unknown, n (%) | 2 (2.1) |
| **First monitored rhythm** (%) | |
| Shockable rhythm | |
| Ventricular fibrillation, n (%) | 69 (71.9) |
| Ventricular tachycardia, n (%) | 1 (1.0) |
| Asystole, n (%) | 9 (9.4) |
| Pulseless electrical activity, n (%) | 13 (13.5) |
| Unknown first rhythm, n (%) | 4 (4.2) |
| **Time from cardiac arrest to event - in minutes** | |
| Start of life support, min, median (min-max) | 0 (0-8) |
| Return of spontaenous circulation, min, median (IQR) | 27.00 (15.75 - 40.75) |
| Administration of epinephrine, min, median (IQR) | 12.00 (9.00 - 15.00) |
| Dose of epinephrine administered, mg, median (IQR) | 3.50 (2.00 - 5.00) |
| **Mode of cooling** | |
| Invasive, n (%) | 71 (74.0) |
| Non-invasive, n (%) | 13 (13.5) |
| Combined, n (%) | 8 (8.3) |
| Unknown, n (%) | 4 (4.2) |

(continued)

| Medical history | |
|---|---|
| Hypertension, n (%) | 61 (63.5) |
| History of smoking, n (%) | 59 (61.5) |
| Diabetes, n (%) | 81 (84.4) |
| Acute myocardial infarction, n (%) | 80 (83.3) |
| COPD, n (%) | 86 (89.6) |
| **Laboratory values at admission** | |
| Potassium mmol/l, median (IQR) | 3.75 (3.32 - 4.40) |
| Calcium mmol/l, median (IQR) | 1.16 (1.11 - 1.25) |
| pO2 mmHg, median (IQR) | 145.50 (90.58 - 300.25) |
| pCO2 mmHg, median (IQR) | 55.10 (44.62 - 63.35) |
| pH, median (IQR) | 7.16 (7.03 -7.24) |
| Bicarbonate mmol/l, median (IQR) | 15.70 (13.12 - 18.28) |
| Base excess mmol/l, median (IQR) | -9.10 (-12.85 - -5.70) |
| Lactate mmol/l, median (IQR) | 7.20 (4.90 - 9.80) |
| Blood glucose mg/dl, median (IQR) | 288 (229 - 349) |
| Hematocrit, %, median (IQR) | 42.50 (38.75 - 45.60) |
| Chloride, mmol/l, median (IQR) | 99.00 (97.00 - 102.00) |
| Creatinine, mg/dl, median (IQR) | 1.27 (1.05 - 1.53) |
| Blood Urea Nitrate, mg/dl, median (IQR) | 15.10 (12.00 - 19.90) |
| Uric Acid mg/dl, median (IQR) | 7.80 (6.70 - 9.10) |
| Total bilirubin, mg/dl, median (IQR) | 0.35 (0.24 - 0.56) |
| Troponin T, ng/L, median (IQR) | 0.07 (0.03 - 0.20) |
| NT-proBNP, pg/ml, median (IQR) | 441 (113 - 1722) |
| S100B, $\mu$g/l, median (IQR) | 0.10 (0.07 - 0.20) |
| Neuron specific enolase, $\mu$g/l, median (IQR) | 30.65 (17.95 - 72.53) |

Table 2. Final multiple reaction monitoring (MRM) method. List of proteins

| UniProt ID | Protein name | Gene name | Uniprot protein entry name |
|---|---|---|---|
| O00299 | Chloride intracellular channel protein 1 | CLIC1 | CLIC1 |
| P00338 | L-lactate dehydrogenase A chain | LDHA | LDHA |
| P00488 | Coagulation factor XIII A chain | F13A1 | F13A |
| P00558 | Phosphoglycerate kinase 1 | PGK1 | PGK1 |
| P02751 | Fibronectin | FN1 | FINC |
| P06733 | Alpha-enolase | ENO1 | ENOA |
| P07195 | L-lactate dehydrogenase B chain | LDHB | LDHB |
| P07737 | Profilin-1 | PFN1 | PROF1 |
| P11142 | Heat shock cognate 71 kDa protein | HSPA8 | HSP7C |

(continued)

| UniProt ID | Protein name | Gene name | Uniprot protein entry name |
|---|---|---|---|
| P18206 | Vinculin | VCL | VINC |
| P23528 | Cofilin-1 | CFL1 | COF1 |
| P63104 | 14-3-3 protein zeta/delta | YWHAZ | 1433Z |
| Q9Y490 | Talin-1 | TLN1 | TLN1 |
| P06702 | Protein S100-A9 | S100A9 | S10A9 |
| P05362 | Intercellular adhesion molecule 1 | ICAM1 | ICAM1 |
| P55290 | Cadherin-13 | CDH13 | CAD 13 |
| P02649 | Apolipoprotein E | APOE | APOE |
| P04040 | Catalase | CAT | CATA |
| P10643 | Complement component C7 | C7 | CO7 |
| P04275 | von Willebrand factor | VWF | VWF |
| P12814 | Alpha-actinin-1 | ACTN1 | ACTN1 |
| Q96KN2 | Beta-Ala-His dipeptidase | CNDP1 | CNDP1 |
| P04406 | Glyceraldehyde-3-phosphate dehydrogenase | GAPDH | G3P |
| P05109 | Protein S100-A8 | S100A8 | S10A8 |

Table 3. Final multiple reaction monitoring (MRM) method. List of peptides and transitions.

| UniProt ID | Peptide sequence | Precursor m/z | Precursor charge | Product m/z | Product charge | Fragment ion | Collision energy |
|---|---|---|---|---|---|---|---|
| O00299 | NSNPALNDNLEK | 664,83 | 2 | 1013,5 3 | 1 | y9 | 19,1 |
| O00299 | NSNPALNDNLEK | 664,83 | 2 | 732,35 | 1 | y6 | 19,1 |
| O00299 | NSNPALNDNLEK | 664,83 | 2 | 507,27 | 2 | y9 | 19,1 |
| O00299 | GFTIPEAFR | 519,27 | 2 | 833,45 | 1 | y7 | 13,9 |
| O00299 | GFTIPEAFR | 519,27 | 2 | 732,4 | 1 | y6 | 13,9 |
| O00299 | GFTIPEAFR | 519,27 | 2 | 619,32 | 1 | y5 | 13,9 |
| O00299 | YLSNAYAR | 479,24 | 2 | 794,42 | 1 | y7 | 12,5 |
| O00299 | YLSNAYAR | 479,24 | 2 | 681,33 | 1 | y6 | 12,5 |
| O00299 | YLSNAYAR | 479,24 | 2 | 594,3 | 1 | y5 | 12,5 |
| P00338 | LVIITAGAR | 457,3 | 2 | 701,43 | 1 | y7 | 11,7 |
| P00338 | LVIITAGAR | 457,3 | 2 | 588,35 | 1 | y6 | 11,7 |
| P00338 | FIIPNVVK | 465,29 | 2 | 669,43 | 1 | y6 | 12 |
| P00338 | FIIPNVVK | 465,29 | 2 | 556,35 | 1 | y5 | 12 |
| P00488 | GTYIPVPIVSELQSGK | 844,47 | 2 | 1253,7 1 | 1 | y12 | 25,6 |
| P00488 | GTYIPVPIVSELQSGK | 844,47 | 2 | 1057,5 9 | 1 | y10 | 25,6 |
| P00488 | STVLTIPEIIIK | 663,92 | 2 | 1039,6 8 | 1 | y9 | 19,1 |
| P00488 | STVLTIPEIIIK | 663,92 | 2 | 926,59 | 1 | y8 | 19,1 |

(continued)

| UniProt ID | Peptide sequence | Precursor m/z | Precursor charge | Product m/z | Product charge | Fragment ion | Collision energy |
|---|---|---|---|---|---|---|---|
| P00488 | STVLTIPEIIIK | 663,92 | 2 | 712,46 | 1 | y6 | 19,1 |
| P00558 | ACANPAAGSVILLENLR | 884,97 | 2 | 1352,79 | 1 | y13 | 27,1 |
| P00558 | ACANPAAGSVILLENLR | 884,97 | 2 | 1184,7 | 1 | y11 | 27,1 |
| P00558 | ACANPAAGSVILLENLR | 884,97 | 2 | 1113,66 | 1 | y10 | 27,1 |
| P02751 | DLQFVEVTDVK | 646,84 | 2 | 936,5 | 1 | y8 | 18,5 |
| P02751 | DLQFVEVTDVK | 646,84 | 2 | 690,37 | 1 | y6 | 18,5 |
| P02751 | DLQFVEVTDVK | 646,84 | 2 | 462,26 | 1 | y4 | 18,5 |
| P02751 | IYLYTLNDNAR | 678,35 | 2 | 1079,55 | 1 | y9 | 19,6 |
| P02751 | IYLYTLNDNAR | 678,35 | 2 | 966,46 | 1 | y8 | 19,6 |
| P02751 | IYLYTLNDNAR | 678,35 | 2 | 589,27 | 1 | y5 | 19,6 |
| P06733 | YISPDQLADLYK | 713,37 | 2 | 1149,58 | 1 | y10 | 20,9 |
| P06733 | YISPDQLADLYK | 713,37 | 2 | 1062,55 | 1 | y9 | 20,9 |
| P06733 | VNQIGSVTESLQACK | 817,41 | 2 | 1179,57 | 1 | y11 | 24,6 |
| P06733 | VNQIGSVTESLQACK | 817,41 | 2 | 936,45 | 1 | y8 | 24,6 |
| P06733 | VNQIGSVTESLQACK | 817,41 | 2 | 506,24 | 1 | y4 | 24,6 |
| P07195 | FIIPQIVK | 479,31 | 2 | 697,46 | 1 | y6 | 12,5 |
| P07195 | FIIPQIVK | 479,31 | 2 | 584,38 | 1 | y5 | 12,5 |
| P07737 | SSFYVNGLTLGGQK | 735,88 | 2 | 986,56 | 1 | y10 | 21,7 |
| P07737 | SSFYVNGLTLGGQK | 735,88 | 2 | 887,49 | 1 | y9 | 21,7 |
| P07737 | SSFYVNGLTLGGQK | 735,88 | 2 | 773,45 | 1 | y8 | 21,7 |
| P07737 | STGGAPTFNVTVTK | 690,36 | 2 | 1191,64 | 1 | y12 | 20,1 |
| P07737 | STGGAPTFNVTVTK | 690,36 | 2 | 1006,56 | 1 | y9 | 20,1 |
| P07737 | STGGAPTFNVTVTK | 690,36 | 2 | 503,78 | 2 | y9 | 20,1 |
| P11142 | DAGTIAGLNVLR | 600,34 | 2 | 855,54 | 1 | y8 | 16,8 |
| P11142 | DAGTIAGLNVLR | 600,34 | 2 | 742,46 | 1 | y7 | 16,8 |
| P11142 | DAGTIAGLNVLR | 600,34 | 2 | 671,42 | 1 | y6 | 16,8 |
| P18206 | AVAGNISDPGLQK | 635,34 | 2 | 744,39 | 1 | y7 | 18,1 |
| P18206 | AVAGNISDPGLQK | 635,34 | 2 | 542,33 | 1 | y5 | 18,1 |

(continued)

| UniProt ID | Peptide sequence | Precurs or m/z | Precurs or charge | Produ ct m/z | Produ ct charg e | Fragme nt ion | Collisio n energy |
|---|---|---|---|---|---|---|---|
| P18206 | SFLDSGYR | 472,73 | 2 | 710,35 | 1 | y6 | 12,2 |
| P18206 | SFLDSGYR | 472,73 | 2 | 597,26 | 1 | y5 | 12,2 |
| P18206 | SFLDSGYR | 472,73 | 2 | 482,24 | 1 | y4 | 12,2 |
| P23528 | AVLFCLSEDK | 591,3 | 2 | 1011,4 8 | 1 | y8 | 16,5 |
| P23528 | AVLFCLSEDK | 591,3 | 2 | 898,4 | 1 | y7 | 16,5 |
| P63104 | SVTEQGAELSNEE R | 774,86 | 2 | 1132,5 2 | 1 | y10 | 23,1 |
| P63104 | SVTEQGAELSNEE R | 774,86 | 2 | 1004,4 6 | 1 | y9 | 23,1 |
| P63104 | SVTEQGAELSNEE R | 774,86 | 2 | 634,28 | 1 | y5 | 23,1 |
| Q9Y490 | GLAGAVSELLR | 543,32 | 2 | 915,53 | 1 | y9 | 14,8 |
| Q9Y490 | GLAGAVSELLR | 543,32 | 2 | 844,49 | 1 | y8 | 14,8 |
| Q9Y490 | GLAGAVSELLR | 543,32 | 2 | 617,36 | 1 | y5 | 14,8 |
| P06702 | LGHPDTLNQGEFK | 485,91 | 3 | 722,35 | 1 | y6 | 12,7 |
| P06702 | LGHPDTLNQGEFK | 485,91 | 3 | 480,25 | 1 | y4 | 12,7 |
| P05362 | LLGIETPLPK | 540,84 | 2 | 854,5 | 1 | y8 | 14,7 |
| P05362 | LLGIETPLPK | 540,84 | 2 | 684,39 | 1 | y6 | 14,7 |
| P05362 | DLEGTYLCR | 563,76 | 2 | 769,37 | 1 | y6 | 15,5 |
| P05362 | DLEGTYLCR | 563,76 | 2 | 611,3 | 1 | y4 | 15,5 |
| P05362 | DLEGTYLCR | 563,76 | 2 | 448,23 | 1 | y3 | 15,5 |
| P55290 | SIWSPILIPENQR | 782,96 | 2 | 1166,6 5 | 1 | y10 | 23,4 |
| P55290 | SIWSPILIPENQR | 782,96 | 2 | 1079,6 2 | 1 | y9 | 23,4 |
| P55290 | SIWSPILIPENQR | 782,96 | 2 | 643,32 | 1 | y5 | 23,4 |
| P02649 | SELEEQLTPVAEE TR | 865,93 | 2 | 1015,5 4 | 1 | y9 | 26,4 |
| P02649 | SELEEQLTPVAEE TR | 865,93 | 2 | 902,46 | 1 | y8 | 26,4 |
| P02649 | SELEEQLTPVAEE TR | 865,93 | 2 | 801,41 | 1 | y7 | 26,4 |
| P02649 | AATVGSLAGQPLQ ER | 749,4 | 2 | 898,47 | 1 | y8 | 22,2 |
| P02649 | AATVGSLAGQPLQ ER | 749,4 | 2 | 827,44 | 1 | y7 | 22,2 |
| P02649 | AATVGSLAGQPLQ ER | 749,4 | 2 | 642,36 | 1 | y5 | 22,2 |
| P04040 | FNTANDDNVTQVR | 747,35 | 2 | 1131,5 4 | 1 | y10 | 22,1 |
| P04040 | FNTANDDNVTQVR | 747,35 | 2 | 1060,5 | 1 | y9 | 22,1 |
| P04040 | FNTANDDNVTQVR | 747,35 | 2 | 831,43 | 1 | y7 | 22,1 |

(continued)

| UniProt ID | Peptide sequence | Precursor m/z | Precursor charge | Product m/z | Product charge | Fragment ion | Collision energy |
|---|---|---|---|---|---|---|---|
| P10643 | SSGWHFWK | 523,77 | 2 | 872,48 | 1 | y7 | 14,1 |
| P10643 | SSGWHFWK | 523,77 | 2 | 629,38 | 1 | y5 | 14,1 |
| P10643 | SCVGETTESTQCEDEELEHLR | 837,02 | 3 | 1329,5 7 | 1 | y10 | 25,3 |
| P10643 | SCVGETTESTQCEDEELEHLR | 837,02 | 3 | 425,26 | 1 | y3 | 25,3 |
| P04275 | LSEAEFEVLK | 582,81 | 2 | 1051,5 3 | 1 | y9 | 16,2 |
| P04275 | LSEAEFEVLK | 582,81 | 2 | 835,46 | 1 | y7 | 16,2 |
| P04275 | LSEAEFEVLK | 582,81 | 2 | 764,42 | 1 | y6 | 16,2 |
| P04275 | EGGPSQIGDALGFAVR | 787,4 | 2 | 1018,5 7 | 1 | y10 | 23,5 |
| P04275 | EGGPSQIGDALGFAVR | 787,4 | 2 | 905,48 | 1 | y9 | 23,5 |
| P04275 | EGGPSQIGDALGFAVR | 787,4 | 2 | 549,31 | 1 | y5 | 23,5 |
| P12814 | DDPLTNLNTAFDVAEK | 881,93 | 2 | 994,48 | 1 | y9 | 26,9 |
| P12814 | DDPLTNLNTAFDVAEK | 881,93 | 2 | 880,44 | 1 | y8 | 26,9 |
| P12814 | DDPLTNLNTAFDVAEK | 881,93 | 2 | 708,36 | 1 | y6 | 26,9 |
| Q96KN2 | SWLIPLGAVDDGEHSQNEK | 703,03 | 3 | 1385,5 9 | 1 | y13 | 20,5 |
| Q96KN2 | SWLIPLGAVDDGEHSQNEK | 703,03 | 3 | 1158,4 7 | 1 | y10 | 20,5 |
| Q96KN2 | SWLIPLGAVDDGEHSQNEK | 703,03 | 3 | 1043,4 4 | 1 | y9 | 20,5 |
| Q96KN2 | WNYIEGTK | 505,75 | 2 | 824,41 | 1 | y7 | 13,4 |
| G296 KN2 | WNYIEGTK | 505,75 | 2 | 710,37 | 1 | y6 | 13,4 |
| P04406 | GALQNIIPASTGAAK | 706,4 | 2 | 815,46 | 1 | y9 | 20,6 |
| P04406 | GALQNIIPASTGAAK | 706,4 | 2 | 702,38 | 1 | y8 | 20,6 |
| P05109 | LLETECPQYIR | 711,36 | 2 | 1195,5 4 | 1 | y9 | 20,8 |
| P05109 | LLETECPQYIR | 711,36 | 2 | 836,41 | 1 | y6 | 20,8 |
| global standard s | VLETKSLYVR | 403,24 | 3 | 554,82 | 2 | y9 | 9,7 |
| global standard s | VLETK[+42]SLYVR | 625,36 | 2 | 908,52 | 1 | y7 | 17,7 |
| global standard s | TTPAVLDSDGSYFLYSK | 932,45 | 2 | 831,41 | 2 | y15 | 28,8 |

Table 4. (Log2) and interquartile ranges (IQR) of selected biomarker candidates in plasma samples of the validation cohort measured with multiple reaction monitoring analysis.

| UniProt ID | Protein name | Median intensity (Log2) negative neurological outcome | IQR negative neurological outcome | Median intensity (Log2) positive neurological outcome | IQR positive neurological outcome |
|---|---|---|---|---|---|
| P63104 | 14-3-3 protein zeta/delta | 9,59 | 8,24 - 10,36 | 8,34 | 7,07-9,48 |
| P12814 | Alpha-actinin-1 | 8,13 | 7,42 - 8,66 | 7,80 | 6,96-8,65 |
| P02649 | Apolipoprotein E | 16,34 | 15,86 16,85 | 16,36 | 15,69-16,84 |
| P55290 | Cadherin-13 | 9,49 | 8,98 - 9,87 | 9,65 | 9,18-10,02 |
| P04040 | Catalase | 8,36 | 7,61 - 8,81 | 7,91 | 7,63-8,69 |
| O00299 | Chloride intracellular channel protein 1 | 10,63 | 9,55 - 11,46 | 9,83 | 9,06-10,98 |
| Q96KN2 | Beta-Ala-His dipeptidase | 9,94 | 9,06 - 10,45 | 9,98 | 9,61-10,32 |
| P10643 | Complement component C7 | 12,10 | 11,41 - 12,43 | 12,13 | 11,66-12,38 |
| P23528 | Cofilin-1 | 10,85 | 9,68 - 11,52 | 9,80 | 8,26-11,09 |
| P06733 | Alpha-enolase | 9,18 | 8,39 - 10,01 | 8,44 | 7,37-9,19 |
| P00488 | Coagulation factor XIII A chain | 11,10 | 10,63 - 11,67 | 11,15 | 10,76-11,78 |
| P02751 | Fibronectin | 14,09 | 13,37 - 14,69 | 14,19 | 13,1-14,63 |
| P04406 | Glyceraldehyde-3-phosphate dehydrogenase | 12,22 | 11,2-13,47 | 11,36 | 10,26-12,59 |
| P11142 | Heat shock cognate 71 kDa protein | 10,51 | 9,94-11,33 | 10,12 | 8,83-10,67 |
| P05362 | Intercellular adhesion molecule 1 | 11,16 | 10,73 - 11,48 | 11,24 | 10,81-11,47 |
| P00338 | L-lactate dehydrogenase A chain | 12,21 | 11,58 - 12,88 | 11,83 | 11,15-12,55 |
| P07195 | L-lactate dehydrogenase B chain | 13,91 | 12,83 - 14,69 | 13,43 | 12,68-14,96 |
| P00558 | Phosphoglycerate kinase 1 | 8,11 | 7,55 - 8,88 | 7,87 | 7,07-8,44 |
| P07737 | Profilin-1 | 10,60 | 9,79 - 11,66 | 9,88 | 8,87-11,18 |
| P05109 | Protein S100-A8 | 11,99 | 11,28 - 12,62 | 11,43 | 10,95-12,21 |
| P06702 | Protein S100-A9 | 13,97 | 13,32 - 14,5 | 13,59 | 13,03-13,96 |
| Q9Y490 | Talin-1 | 11,26 | 10,19 - 12,07 | 10,56 | 8,57-11,67 |

(continued)

| UniProt ID | Protein name | Median intensity (Log2) negative neurological outcome | IQR negative neurologic al outcome | Median intensity (Log2) positive neurological outcome | IQR positive neurologic al outcome |
|---|---|---|---|---|---|
| P1820 6 | Vinculin | 11,82 | 11,08 - 12,56 | 11,42 | 10,69-12,15 |
| P0427 5 | von Willebrand factor | 13,82 | 13,44 - 14,06 | 13,59 | 13,37-14,04 |

Table 5. Univariate regression analysis of all biomarker candidates to predict negative neurological outcome.

| UniProt ID[a] | Protein name | Coefficient | Odds ratio | 95% CI for OR |
|---|---|---|---|---|
| P63104 | 14-3-3 protein zeta/delta | 0.4757 | 1.6092 | 1.1971 - 2.2382 |
| P12814 | Alpha-actinin-1 | 0.3307 | 1.3920 | 0.9609 - 2.0694 |
| P06733 | Alpha-enolase | 0.6529 | 1.9210 | 1.2942 - 3.0044 |
| P02649 | Apolipoprotein E | -0.0307 | 0.9697 | 0.5915 - 1.5609 |
| Q96KN2 | Beta-Ala-His dipeptidase | -0.0352 | 0.9654 | 0.5907 - 1.5460 |
| P55290 | Cadherin-13 | -0.1486 | 0.8619 | 0.4592 - 1.4147 |
| P04040 | Catalase | 0.2030 | 1.2251 | 0.8236 - 1.8741 |
| 000299 | Chloride intracellular channel protein 1 | 0.3891 | 1.4756 | 1.0099 - 2.2169 |
| P00488 | Coagulation factor XIII A chain | 0.2024 | 1.2244 | 0.7627 - 2.0094 |
| P23528 | Cofilin-1 | 0.3959 | 1.4857 | 1.1208 - 2.0391 |
| P10643 | Complement component C7 | -0.0070 | 0.9930 | 0.5578 - 1.7050 |
| P02751 | Fibronectin | 0.0516 | 1.0529 | 0.7198 - 1.5238 |
| P04406 | Glyceraldehyde-3-phosphate dehydrogase | 0.3927 | 1.4810 | 1.0750 - 2.0986 |
| P11142 | Heat shock cognate 71 kDa protein | 0.6375 | 1.8917 | 1.2507 - 3.0154 |
| P05362 | Intercellular adhesion molecule 1 | 0.0901 | 1.0943 | 0.6353 - 1.8424 |
| P00338 | L-lactate dehydrogenase A chain | 0.4638 | 1.5901 | 1.0234 - 2.6641 |
| P07195 | L-lactate dehydrogenase B chain | 0.2027 | 1.2247 | 0.8921 - 1.7279 |

(continued)

| UniProt ID[a] | Protein name | Coefficient | Odds ratio | 95% CI for OR |
|---|---|---|---|---|
| P00558 | Phosphoglycerate kinase 1 | 0.5121 | 1.6689 | 1.0580 - 2.8926 |
| P07737 | Profilin-1 | 0.4148 | 1.5140 | 1.0911 - 2.1646 |
| P05109 | Protein S100-A8 | 0.2526 | 1.2874 | 0.8872 - 1.9117 |
| P06702 | Protein S100-A9 | 0.2709 | 1.3111 | 0.8782 - 2.0198 |
| Q9Y490 | Talin-1 | 0.3234 | 1.3818 | 1.0518 - 1.8500 |
| P18206 | Vinculin | 0.4858 | 1.6256 | 1.0312 - 2.6682 |
| [a] Swiss-Prot (UniProt) accession numbers are provided. | | | | |

Table 6. Model 1, linear prediction model for negtive neurological outcome consisting of established clinical predictors of neurological outcome

| Parameters | UniProt ID | Coefficient | Absolute Standardized coefficient |
|---|---|---|---|
| Intercept | | 0.48465 | |
| Time from cardiac arrest to start of life support (not immediate vs. immediate) | | 0.88147 | 0.39245 |
| Age (per year) | | 0.02693 | 0.36597 |
| Shockable rhythm (yes vs. no) | | -0.80759 | 0.36436 |
| Maximum absolute difference of pH to 7.3pH within the first 24 hours | | 1.14039 | 0.19586 |
| Hemoglobin (g/dl) | | -0.05861 | 0.10948 |
| Time from cardiopulmonary resuscitation to the return of spontaneous circulation (per doubling, minutes)[b] | | -0.07873 | 0.09728 |
| Maximum serum lactate within the first 24 hours (mmol/L) | | 0.00909 | 0.03826 |

Table 7. Model 2, prediction model for negative neurological outcome consisting of potential predictors of neurological outcome estimated by a LASSO logistic regression model

| Parameters | UniProt ID | Gene name | Coefficient | Absolute Standardized coefficient | Bootstrap inclusion frequency |
|---|---|---|---|---|---|
| Intercept | | | 1.04935 | | a |
| Age (per year) | | | 0.02693 | 0.36597 | a |
| Shockable rhythm (yes vs. no) | | | -0.80759 | 0.36436 | a |
| Time from cardiac arrest to start of life support (not immediate vs. immediate) | | | 0.88147 | 0.39245 | a |

(continued)

| Parameters | UniProt ID | Gene name | Coefficient | Absolute Standardized coefficient | Bootstrap inclusion frequency |
|---|---|---|---|---|---|
| Hemoglobin (g/dl) | | | -0.05861 | 0.10948 | a |
| Time from cardiopulmonary resuscitation to the return of spontaneous circulation (per doubling, minutes)b | | | -0.07873 | 0.09728 | a |
| Maximum serum lactate within the first 24 hours (mmol/L) | | | 0.00909 | 0.03826 | a |
| Maximum absolute difference of pH to 7.3pH within the first 24 hours | | | 1.14039 | 0.19586 | a |
| S-100B (per doubling)b | P04271 | S100B | 0.19187 | 0.36826 | 81.9 |
| Adrenalin administrated during Cardiopulmonary | | | 0.00000 | 0.00000 | 41.3 |
| resuscitation (mg) | | | | | |
| Neuron-specific enolase (per doubling)a | P09104 | ENO2 | 0.00000 | 0.00000 | 36.5 |
| Sex (female vs. male) | | | 0.00000 | 0.00000 | 27.5 |
| Unwitnessed Cardiac arrest (yes vs. no) | | | 0.00000 | 0.00000 | 15.2 |
| a These predictors were always included and not subjected to variable selection, b Measured by electrochemiluminescence immunoassay. | | | | | |

Table 8. Model 3, proteomics-enriched prediction model for negative neurological outcome estimated by LASSO logistic regression.

| Parameters | UniProt ID | Gene name | Coefficient | Absolute Standardized coefficient | Bootstrap inclusion frequency |
|---|---|---|---|---|---|
| Intercept | | | -1.86718 | | a |
| Age (per year) | | | 0.02693 | 0.36597 | a |
| Shockable rhythm (yes vs. no) | | | -0.80759 | 0.36436 | a |
| Time from cardiac arrest to start of life support (not immediate vs. immediate) | | | 0.88147 | 0.39245 | a |
| Hemoglobin (g/dl) | | | -0.05861 | 0.10948 | a |
| Time from cardiopulmonary resuscitation to the return of spontaneous circulation (per doubling, minutes)b | | | -0.07873 | 0.09728 | a |
| Maximum serum lactate within the first 24 hours (mmol/L) | | | 0.00909 | 0.03826 | a |
| Maximum absolute difference of pH to 7.3pH within the first 24 | | | 1.14039 | 0.19586 | a |
| hours | | | | | |

(continued)

| Parameters | UniProt ID | Gene name | Coefficient | Absolute Standardized coefficient | Bootstrap inclusion frequency |
|---|---|---|---|---|---|
| S-100B (per doubling)[b] | P04271 | S100B | 0.10194 | 0.19565 | 79.8 |
| Alpha-enolase (per doubling)[d] | P06733 | ENO1 | 0.16576 | 0.21045 | 55.1 |
| 14-3-3 protein zeta/delta (per doubling)[d] | P63104 | YWHAZ | 0.09157 | 0.14942 | 60.1 |
| Cofilin-1 (per doubling)[d] | P23528 | CFL1 | 0.03819 | 0.06493 | 58.5 |
| [a] These predictors were always included and not subjected to variable selection. [b] log2(x+1) transformation was applied. [c] Measured by electrochemiluminescence immunoassay. [d] Measured by Targeted LC-MRM analysis. Swiss-Prot (UniProt) accession numbers are provided. | | | | | |

Table 9. Model 4, proteomics-enriched prediction model for negative neurological outcome estimated by elastic-net logistic regression

| Parameters | UniProt ID | Gene name | Coefficient | Absolute Standardized coefficient | Bootstrap inclusion frequency |
|---|---|---|---|---|---|
| Intercept | | | -1.72579 | | a |
| Age (per year) | | | 0.02693 | 0.36597 | a |
| Shockable rhythm (yes vs. no) | | | -0.80759 | 0.36436 | a |
| Time from cardiac arrest to start of life support (not immediate vs. immediate) | | | 0.88147 | 0.39245 | a |
| Hemoglobin (g/dl) | | | -0.05861 | 0.10948 | a |
| Time from cardiopulmonary resuscitation to the return of spontaneous circulation (per doubling, minutes)[b] | | | -0.07873 | 0.09728 | a |
| Maximum serum lactate within the first 24 hours (mmol/L) | | | 0.00909 | 0.03826 | a |
| Maximum absolute difference of pH to 7.3pH within the first 24 hours | | | 1.14039 | 0.19586 | a |
| S-100B (per doubling)[b] | P04271 | S100B | 0.07509 | 0.14411 | 86.5 |
| Alpha-enolase (per doubling)[b] | P06733 | ENO1 | 0.11104 | 0.14098 | 81.4 |
| 14-3-3 protein zeta/delta (per doubling)[c] | P63104 | YWHAZ | 0.07876 | 0.12851 | 78.9 |
| Cofilin-1 (per doubling)[b] | P23528 | CFL1 | 0.05262 | 0.08946 | 81.5 |
| Heat shock cognate 71 kDa protein (per doubling)[b] | P11142 | HSPA8 | 0.02181 | 0.02595 | 46.1 |
| [a] These predictors were always included and not subjected to variable selection. [b] log2(x+1) transformation was applied. [c] Measured by electrochemiluminescence immunoassay. [d] Measured by Targeted LC-MRM analysis. Swiss-Prot (UniProt) accession numbers are provided. | | | | | |

Table 10. Summary of models 1-4 for predicting negative neurological outcome using established and new biomarkers

| Model | Method | Predictors | C-index | Explained variation in % | p-value for 'added value' |
|---|---|---|---|---|---|
| Model 1 | Ridge regression | Fixed: Age, shockable rhythm, time from OHCA to CPR (not immediate vs. immediate), time from CPR to ROSC, hemoglobin, lactate, pH | 0.67 | 8.2% | 0.002[a] |
| Model 2 | Lasso regression | Fixed: Linear predictor from M1 Candidates: doses of administrated adrenalin, sex, witnessed OHCA, S100B, NSE Selected: S100B | 0.68 | 10.0% | 0.147[b] |
| Model 3 | Lasso regression | Fixed: Linear predictor from M1 Candidates: doses of administrated adrenalin, sex, witnessed OHCA, S100B, NSE, all proteins measured by Targeted LC-MRM analysis Selected: S100B, Alpha-enolase, 14-3-3 protein zeta/delta, Cofilin-1 | 0.70 | 12.1% | 0.025[c] |
| Model 4 | Elastic-net regression | Fixed: Linear predictor from M1 Candidates: Doses of administrated adrenalin, sex, witnessed OHCA, S100B, NSE, all proteins measured by Targeted LC-MRM analysis Selected: S-100B, Alpha-enolase, 14-3-3 protein zeta/delta, Cofilin-1, Heat shock cognate 71 kDa protein | 0.70 | 11.9% | 0.019[c] |

[a] compared to null model
[b] compared to Model 1
[c] compared to Model 2

Table 11. Calculated percentage changes relative to the median levels of each biomarker that allow prediction of a positive or negative neurological outcome.

| Gene name | Percentage change for positive neurological outcome | Percentage change for negative neurological outcome |
|---|---|---|
| Alpha-enolase | 31% (sensitivity: 0.15, specificy: 0.98) | 344% (sensitivity: 0.23, specificy: 1) |
| Heat shock cognate 71 kDa protein | 26% (sensitivity: 0.06, specificy: 1) | 277% (sensitivity: 0.23, specificy: 1) |
| 14-3-3 protein zeta/delta | 24% (sensitivity: 0.15, specificy: 0.98) | 372% (sensitivity: 0.29, specificy: 0.97) |
| Cofilin-1 | 18% (sensitivity: 0.12, specificy: 1) | 418% (sensitivity: 0.21, specificy: 0.97) |

**Example 2** - **Results**

*Baseline characteristics:* One-hundred OHCA patients wre included with a median age of 58 years (IQR, 49-69) admitted to the emergency department of the Medical University of Vienna between October 2013 and May 2016. Four patients were excluded because of poor specimen quality. Hence, the final study population consisted of 96 patients, of whom 63 patients (66 percent) had a poor neurological outcome at discharge from the ICU. CPC 1 was recorded in 26 patients (27%), CPC 2 in 7 (7%), CPC 3 in 24 (23%), CPC 4 in 16 (15%), and CPC 5 in 23 (22%) of OHCA survivors. Detailed baseline characteristics are displayed in Table 1.

*Identification of biomarker candidates:* The flow chart of proteomic analyses is illustrated in Figure 1. A total of 299 proteins were identified in plasma samples from the sentinel population by proteomic shotgun analyses. After crosschecking with the proteome of 10 independent brain autopsy samples (4595 proteins), 135 proteins were detected within plasma as well as brain tissue samples. Of these proteins, 60 proteins fulfilled selection criteria and were taken into consideration for MRM assay development. Finally, a MRM method was established as potential prognostic signature, consisting of 24 proteins represented by 40 proteotypic peptides and 100 optimized mass transitions in the triple-quadrupol MS that was applied for measurement in the verification cohort (Table 3). Protein abundance values of MRM analyses are summarized in Table 4.

*Prognostic model:* The results of a univariate regression analysis of each of the 24 biomarker candidates to predict poor neurological outcome are outlined in Table 5. To explore the best multivariable combination of clinical parameters and biomarkers to predict neurological outcome in OHCA patients, a multi-stage statistical approach was applied. M1, including all well-established neurological outcome predictors, achieved a discriminative ability (cross-validated c-index) of 0.67. This c-index increased to 0.68 by the variable selection process of M2, which added S100-B. M3 additionally selected a multivariable combination of the proteomically measured biomarker candidates (n=24) and yielded a c-index of 0.70 (Table 9). This optimal combination, consisting of alpha-enolase, 14-3-3 protein zeta/delta and cofilin-1, resulted in a significant improvement in neurological outcome prediction compared to M2 (p=0.025 for added value). M4, which employed elastic-net as a less restrictive variable selection procedure, additionally selected Heat shock cognate 71 kDa protein (HSPA8), yielding similar performance (c-index= 0.70; p= 0.019 for added value compared to M2, Table 3). Explained variation of the outcome could be improved from 8.2% in M1 to 10.0% in M2 and to 12.2% in M3 and to 11.9% in M4 (Table 4). Figure 2 depicts the agreement between predicted and observed risk for poor outcome and confirms a reasonable calibration of M3 and M4. The Tables 6 and 7 outline the estimated prediction models M1 and M2.

## Example 3 - Summary and discussion

The present study identified a comprehensive panel of novel biomarkers for the prediction of poor neurological outcome in OHCA survivors using a three-step proteomics strategy. The implementation of proteomically identified alpha-enolase, and optionally further one or more of 14-3-3 protein zeta/delta cofilin-1 and HSPA8 into the clinical model resulted in a significant improvement of established neurological outcome prediction. These biomarkers were carefully retrieved out of a total of 299 plasma proteins measured in successfully resuscitated patients 48 hours after cardiac arrest using a proteomics shotgun analysis, crosschecked in brain tissue samples and verified using targeted proteomic analyses in combination with a stepwise statistical approach including penalization for the number of potential proteomic candidates and optimism-corrected assessment of discrimination and calibration.

The central nervous system is protected by the blood-brain barrier, composing of brain vascular endothelial cells and associated astrocytes, that restricts molecular exchange between the brain and blood vessels to regulate the cerebral homeostasis. Nevertheless, the detection of brain-derived proteins in circulating blood is feasible in patients undergoing cardiac arrest, as hypoxia triggers disruption of the blood-brain barrier. In the present study, all identified biomarkers were not only detected in circulating plasma but also in brain tissue samples. Even though these proteins are not specifically expressed by brain tissue, a causal relationship with neurological recovery is supported by their biological functions and processes in which they are implicated. In addition to that, the prior art biomarkers NSE and S100B are not specific to neuronal damage as well and have been found produced by extra-central nervous system sources.

In the art prediction of neurological outcome in OHCA survivors remains a clinical challenge. Neurophysiological tests are recommended by current guidelines to be performed in individuals not regaining consciousness within two days after the event. Technically demanding, these tests are not readily available in the majority of hospitals. Early, reliable prognosis within the first days following ROSC is often impossible based on currently available models including clinical assessment and biomarkers (e.g. NSE, S100) - leaving clinicians and relatives in uncertainty.

This study considerably extends the limited knowledge in cognitive outcome prediction of OHCA survivors using *inter alia* a state-of-the-art large-scale MS based proteomics strategy. For the first time alpha-enolase, 14-3-3 protein zeta/delta, and/or cofilin-1 were revealed as additional robust predictors, for example,e to be used in a multivariable model of poor or good neurological outcome in an OHCA study population. In fact, the incorporation of alpha-enolase, 14-3-3 protein zeta/delta, cofilin-1 and/or HSPA8 into a multimarker model provided a clear improvement in predictive accuracy and discriminatory power for poor neurological outcome.

The poor neurological outcome in 66% of our OHCA study population, which is in the range of other studies highlights the continuing clinical need to estimate neurological recovery early after cardiac arrest. A timely and accurate prediction would essentially help clinicians in their decision-making processes for evaluating further therapeutic strategies or withdrawal of therapies. The proteomics-enriched prediction model described herein consists exclusively of plasma biomarkers and cardiopulmonary resuscitation (CPR) related factors and could therefore be reliably calculated within 48 hours after cardiac arrest. By using this model, prediction is advantageously delayed due to outstanding examinations that are not available 2417.

Therapeutic hypothermia and concomitant administration of drugs for sedation and muscle paralysis after cardiac arrest crucially complicates and delays prognostication by affecting not only the assessment of clinical reactivity but also the interpretation of neurophysiological tests. A valid prognostication is further delayed as therapeutic hypothermia prolongs the action of both sedative agents and muscle relaxants by slowing their clearance. Biomarkers are presumably not affected by sedation and may therefore provide more reliable objective prognostic information in the early post-resuscitation phase. As currently available biomarkers of neurological recovery are greatly limited in their prognostic value, the four biomarkers revealed in connection with the present invention represent a valuable addition to established risk predictors.

In summary, the study presented in the examples created a superior early prediction model for poor or good neurological outcome in OHCA survivors. A significant improvement in neurological outcome prediction was found based on alpha-enolase, 14-3-3 protein zeta/delta, cofilin-1 and/or HSPA8, in particular when included into a multimarker predictive model along with established risk factors. Each of the biomarkers did not only refine current risk stratification models but may also reflect important pathophysiological pathways undergoing during cerebral ischemia.

**Claims**

1. Method for predicting the neurological outcome of a patient after cardiac arrest, the method comprising the step(s) of:

    (I)

        a) determining the level of alpha-enolase in a biological sample that has been obtained from a patient after cardiac arrest; and
        b) comparing the level of alpha-enolase obtained in (a) with the median or mean level of alpha-enolase in biological samples from patients with positive neurological outcome after cardiac arrest,
        wherein said patient is diagnosed as having
        a negative neurological outcome if the alpha-enolase level is increased by at least 100% over the median or mean level of alpha-enolase, and/or
        a positive neurological outcome if the alpha-enolase level is below 150% as compared to the median or mean level of alpha-enolase; or

    (II)
    determining the level of alpha-enolase in a biological sample that has been obtained from a patient after cardiac arrest,

        wherein said patient is diagnosed as having
        a negative neurological outcome if the alpha-enolase level is above 50 $\mu$g/L, and/or
        a positive neurological outcome if the alpha-enolase level is below 50 $\mu$g/L.

2. The method of claim 1, comprising prior to step (a) the step of determining the median or mean level of alpha-enolase in biological samples from patients with positive neurological outcome after cardiac arrest.

3. The method of claim 1 or 2, further comprising

    (I')

        c) determining the level of at least one additional biomarker in the biological sample obtained from a patient after cardiac arrest; and
        d) comparing the level of said at least one additional biomarker obtained in (c) with the median or mean level of said at least one additional biomarker in biological samples from patients with positive neurological outcome after cardiac arrest,
        wherein said at least one additional biomarker is selected from the group consisting of the heat shock cognate 71 kDa protein, 14-3-3 protein zeta/delta, and cofilin-1, and
        wherein said patient is diagnosed as having
        a negative neurological outcome if the level of said at least one additional biomarker is increased by at least 100% over the median or mean level of said at least one additional biomarker, and/or
        as having a positive neurological outcome if the level of said at least one additional biomarker is below 150% as compared to the median or mean level of said at least one additional biomarker; or

    (II')
    determining the level of at least one additional biomarker in the biological sample obtained from a patient after cardiac arrest,

wherein said at least one additional biomarker is selected from the group consisting of the heat shock cognate 71 kDa protein, 14-3-3 protein zeta/delta, and cofilin-1, and

wherein said patient is diagnosed as having

a negative neurological outcome if the heat shock cognate 71 kDa protein level is above 490 μg/L, the 14-3-3 protein zeta/delta protein level is above 2.7 mg/L and/or the cofilin-1 protein level is above 180 μg/L, and/or

a positive neurological outcome if the heat shock cognate 71 kDa protein level is below 490 μg/L, the 14-3-3 protein zeta/delta protein level is below 2.7 mg/L and/or the cofilin-1 protein level is below 180 μg/L.

4. The method of claim 3, further comprising prior to step (c) the step of determining the median or mean level of the at least one additional biomarker in biological samples from patients with positive neurological outcome after cardiac arrest.

5. The method of any of claims 1-4, wherein the patients with positive neurological outcome after cardiac arrest are at least 5, preferably at least 10, more preferably at least 20, and most preferably at least 30 patients.

6. The method of any of claims 1-5, wherein the biological sample(s) is/are whole blood, plasma, serum, cerebrospinal fluid or brain tissue.

7. The method of claim 6, wherein the biological sample(s) is/are plasma.

8. The method of any of claims 1-7, wherein patients with a positive neurological outcome are patients having after recovery from the cardiac arrest a Cerebral Performance Category (CPC) score of 1 or 2.

9. The method of any of claims 1-8, wherein the level of alpha-enolase and optionally also the level of the at least one additional biomarker has been/have been/is/are determined by mass spectrometry, enzyme-linked immunosorbent assay (ELISA), electrochemiluminescence assay and/or a radioimmunoassay.

10. The method of any of claims 1-9, wherein the biological sample(s) obtained from patient(s) after cardiac arrest has/have been obtained within 96h, preferably within 72h, more preferably between 24 and 72h and most preferably about 48h after cardiac arrest.

11. The method of any of claims 1-10, further comprising
predicting the neurological outcome after cardiac arrest in the patient on the basis of one or more selected from the age of the patient, shockable rhythm, haemoglobin concentration, the time from cardiac arrest to the start of life support, the time from cardiopulmonary resuscitation to return of spontaneous circulation (ROSC), the maximum serum lactate within 24h after cardiac arrest, the maximum difference to the pH of 7.3 within 24h after cardiac arrest, and the level of the protein S-110B in the sample of the patient.

12. Use of a binding agent which specifically binds to alpha-enolase in a method for predicting the neurological outcome of a patient after cardiac arrest according to claim 1.

**Patentansprüche**

1. Verfahren zur Vorhersage des neurologischen Ergebnisses eines Patienten nach Herzstillstand, wobei das Verfahren den/die Schritt(e) umfasst:

(I)

a) Bestimmung des Levels an Alpha-Enolase in einer biologischen Probe, die vom den Patienten nach dem Herzstillstand entnommen wurde; und
b) Vergleich des in (a) erhaltenen Levels an Alpha-Enolase mit dem medianen oder mittleren Level an Alpha-Enolase in biologischen Proben von Patienten mit positivem neurologischen Ergebnis nach Herzstillstand,
wobei der Patient diagnostiziert wird als habend

ein negatives neurologisches Ergebnis, wenn der Level an Alpha-Enolase um mindestens 100% im Vergleich zum medianen oder mittleren Level an Alpha-Enolase erhöht ist, und/oder

ein positives neurologisches Ergebnis, wenn der Level an Alpha-Enolase unter 150% im Vergleich zum medianen oder mittleren Level an Alpha-Enolase ist; oder

(II)

Bestimmung des Levels an Alpha-Enolase in einer biologischen Probe, die von dem Patienten nach dem Herzstillstand entnommen wurde,

wobei der Patient diagnostiziert wird als habend

ein negatives neurologisches Ergebnis, wenn der Level an Alpha-Enolase über 50 $\mu$g/L ist, und/oder

ein positives neurologisches Ergebnis, wenn der Level an Alpha-Enolase unter 50 $\mu$g/L ist.

2. Verfahren nach Anspruch 1, weiterhin umfassend vor dem Schritt (a) den Schritt der Bestimmung des medianen oder mittleren Levels an Alpha-Enolase in biologischen Proben von Patienten mit positivem neurologischen Ergebnis nach Herzstillstand.

3. Verfahren nach Anspruch 1 oder 2, weiterhin umfassend

(I')

c) Bestimmung des Levels mindestens eines weiteren Biomarkers in der biologischen Probe, die vom den Patienten nach dem Herzstillstand entnommen wurde; und

d) Vergleich des in (c) erhaltenen Levels des mindestens einen weiteren Biomarkers mit dem medianen oder mittleren Level des mindestens einen weiteren Biomarkers in biologischen Proben von Patienten mit positivem neurologischen Ergebnis nach Herzstillstand,

wobei der mindestens eine weitere Biomarker ausgewählt ist aus der Gruppe bestehend aus Hitzeschock erkennendes 71 kDa Protein, 14-3-3 Protein zeta/delta und Cofilin-1, und

wobei der Patient diagnostiziert wird als habend

ein negatives neurologisches Ergebnis, wenn der Level des mindestens einen weiteren Biomarkers um mindestens 100% im Vergleich zum medianen oder mittleren Level des mindestens einen weiteren Biomarkers erhöht ist, und/oder ein positives neurologisches Ergebnis, wenn der Level des mindestens einen weiteren Biomarkers unter 150% im Vergleich zum medianen oder mittleren Level des mindestens einen weiteren Biomarkers ist; oder

(II')

Bestimmung des Levels mindestens eines weiteren Biomarkers in der biologischen Probe, die vom den Patienten nach dem Herzstillstand entnommen wurde,

wobei der mindestens eine weitere Biomarker ausgewählt ist aus der Gruppe bestehend aus Hitzeschock erkennendes 71 kDa Protein, 14-3-3 Protein zeta/delta und Cofilin-1, und

wobei der Patient diagnostiziert wird als habend

ein negatives neurologisches Ergebnis, wenn der Level an Hitzeschock erkennendes 71 kDa Protein über 490 $\mu$g/L, 14-3-3 Protein zeta/delta über 2,7 mg/L und/oder Cofilin-1 über 180 $\mu$g/L ist, und/oder

ein positives neurologisches Ergebnis, wenn der Level an Hitzeschock erkennendes 71 kDa Protein unter 490 $\mu$g/L, 14-3-3 Protein zeta/delta unter 2,7 mg/L und/oder Cofilin-1 unter 180 $\mu$g/L ist.

4. Verfahren nach Anspruch 3, weiterhin umfassend vor dem Schritt (c) den Schritt der Bestimmung des medianen oder mittleren Levels des mindestens einen weiteren Biomarkers in biologischen Proben von Patienten mit positivem neurologischen Ergebnis nach Herzstillstand.

5. Verfahren nach einem der Ansprüche 1 bis 4, wobei die Patienten mit positivem neurologischen Ergebnis nach Herzstillstand mindestens 5, bevorzugt mindestens 10, mehr bevorzugt mindestens 20 und am meisten bevorzugt mindestens 30 Patienten sind.

6. Verfahren nach einem der Ansprüche 1 bis 5, wobei die biologische(n) Probe(n) Vollblut, Plasma, Serum, Liquor cerebrospinalis oder Gehirngewebe ist/sind.

**7.** Verfahren nach Anspruch 6, wobei die biologische(n) Probe(n) Plasma ist/sind.

**8.** Verfahren nach einem der Ansprüche 1 bis 7, wobei die Patienten mit positivem neurologischen Ergebnis Patienten sind, die nach der Erholung vom Herzstillstand einen Cerebral Performance Category (CPC)-Wert von 1 oder 2 zeigen.

**9.** Verfahren nach einem der Ansprüche 1 bis 8, wobei der Level an Alpha-Enolase und gegebenenfalls auch der Level des mindestens einen weiteren Biomarkers mittels Massenspektrometrie, Enzyme-linked Immunosorbent Assay (ELISA), Chemielumineszenzassay und/oder Radioimmunassay bestimmt wurde(n) oder wird/werden.

**10.** Verfahren nach einem der Ansprüche 1 bis 9, wobei die biologischen Probe(n), die vom/von den Patienten nach dem Herzstillstand entnommen wurde(n) innerhalb von 96h, bevorzugt innerhalb von 72h, mehr bevorzugt innerhalb von zwischen 24 und 72h und am meisten bevorzugt nach etwa 48h nach Herzstillstand entnommen wurde(n).

**11.** Verfahren nach einem der Ansprüche 1 bis 10, weiterhin umfassend
Vorhersage des neurologischen Ergebnisses nach Herzstillstand in dem Patienten auf Basis von einem oder mehreren ausgewählt aus Alter des Patienten, defibrillierbarem Rhythmus, Hämoglobinkonzentration, der Zeit zwischen Herzstillstand und Wiederbelebung, Zeit von der Reanimation bis zur Rückkehr der spontanen Zirkulation (ROSC), maximaler Serumlaktat innerhalb von 24h nach Herzstillstand, maximaler Unterschied zum pH von 7,3 innerhalb von 24h nach Herzstillstand und Level des Proteins S-110B in der Probe des Patienten.

**12.** Verwendung eines Bindeagens, das spezifisch an Alpha-Enolase bindet in einem Verfahren zur Vorhersage des neurologischen Ergebnisses eines Patienten nach Herzstillstand nach Anspruch 1.

**Revendications**

**1.** Méthode pour prédire l'issue neurologique d'un patient après un arrêt cardiaque, la méthode comprenant la ou les étapes de:

(I)

a) détermination du niveau d'alpha-énolase dans un échantillon biologique qui a été obtenu auprès d'un patient après un arrêt cardiaque; et
b) comparaison du niveau d'alpha-énolase obtenu en (a) avec le niveau médian ou moyen d'alpha-énolase dans des échantillons biologiques provenant de patients avec une issue neurologique positive après un arrêt cardiaque,
dans laquelle ledit patient est diagnostiqué comme ayant
une issue neurologique négative si le niveau d'alpha-énolase est augmenté d'au moins 100 % par rapport au niveau médian ou moyen d'alpha-énolase, et/ou une issue neurologique positive si le niveau d'alpha-énolase est inférieur à 150 % en comparaison avec le niveau médian ou moyen d'alpha-énolase; ou

(II)

détermination du niveau d'alpha-énolase dans un échantillon biologique qui a été obtenu auprès d'un patient après un arrêt cardiaque,
dans laquelle ledit patient est diagnostiqué comme ayant
une issue neurologique négative si le niveau d'alpha-énolase est supérieur à 50 $\mu$g/l, et/ou
une issue neurologique positive si le niveau d'alpha-énolase est inférieur à 50 $\mu$g/l.

**2.** Méthode selon la revendication 1, comprenant, avant l'étape (a), l'étape de détermination du niveau médian ou moyen d'alpha-énolase dans des échantillons biologiques provenant de patients avec une issue neurologique positive après un arrêt cardiaque.

**3.** Méthode selon la revendication 1 ou 2, comprenant en outre

(I')

c) la détermination du niveau d'au moins un marqueur biologique additionnel dans l'échantillon biologique obtenu auprès d'un patient après un arrêt cardiaque; et

d) la comparaison du niveau dudit au moins un marqueur biologique additionnel obtenu en (c) avec le niveau médian ou moyen dudit au moins un marqueur biologique additionnel dans des échantillons biologiques provenant de patients avec une issue neurologique positive après un arrêt cardiaque,

dans laquelle ledit au moins un marqueur biologique additionnel est sélectionné dans le groupe constitué par la protéine apparentée à la protéine de choc thermique de 71 kDa, la protéine 14-3-3 zêta/delta, et la cofiline 1, et dans laquelle ledit patient est diagnostiqué comme ayant

une issue neurologique négative si le niveau dudit au moins un marqueur biologique additionnel est augmenté d'au moins 100 % par rapport au niveau médian ou moyen dudit au moins un marqueur biologique additionnel, et/ou

une issue neurologique positive si le niveau dudit au moins un marqueur biologique additionnel est inférieur à 150 % en comparaison du niveau médian ou moyen dudit au moins un marqueur biologique additionnel; ou

(II')

la détermination du niveau d'au moins un marqueur biologique additionnel dans l'échantillon biologique qui a été obtenu auprès d'un patient après un arrêt cardiaque,

dans laquelle ledit au moins un marqueur biologique additionnel est sélectionné dans le groupe constitué par la protéine apparentée à la protéine de choc thermique de 71 kDa, la protéine 14-3-3 zêta/delta, et la cofiline 1, et dans laquelle ledit patient est diagnostiqué comme ayant

une issue neurologique négative si le niveau de la protéine de choc thermique de 71 kDa est supérieur à 490 $\mu$g/l, le niveau de la protéine 14-3-3 zêta/delta est supérieur à 2,7 mg/l et/ou le niveau de la protéine cofiline-1 est supérieur à 180 $\mu$g/l, et/ou

une issue neurologique positive si le niveau de la protéine de choc thermique de 71 kDa est inférieur à 490 $\mu$g/l, le niveau de la protéine 14-3-3 zêta/delta est inférieur à 2,7 mg/l et/ou le niveau de la protéine cofiline-1 est inférieur à 180 $\mu$g/l.

4. Méthode selon la revendication 3, comprenant en outre, antérieur à l'étape (c), l'étape de détermination du niveau médian ou moyen de l'au moins un marqueur biologique additionnel dans des échantillons biologiques provenant de patients avec une issue neurologique positive après un arrêt cardiaque.

5. Méthode selon l'une quelconque des revendications 1 à 4, dans laquelle les patients avec une issue neurologique positive après un arrêt cardiaque sont au moins 5, de préférence au moins 10, mieux encore au moins 20, et de manière préférée entre toutes au moins 30 patients.

6. Méthode selon l'une quelconque des revendications 1 à 5, dans laquelle le ou les échantillons biologiques sont du sang entier, du plasma, du sérum, du liquide céphalo-rachidien ou du tissu cérébral.

7. Méthode selon la revendication 6, dans laquelle le ou les échantillons biologiques sont du plasma.

8. Méthode selon l'une quelconque des revendications 1 à 7, dans laquelle les patients avec une issue neurologique positive sont des patients ayant, après avoir récupéré de l'arrêt cardiaque, une note de catégorie de performance cérébrale (CPC) de 1 ou 2.

9. Méthode selon l'une quelconque des revendications 1 à 8, dans laquelle le niveau d'alpha-énolase et optionnellement aussi le niveau de l'au moins un marqueur biologique additionnel ont été / sont déterminés par spectrométrie de masse, immunodosage enzymatique (ELISA), dosage par électrochimioluminescence et/ou radio-immunodosage.

10. Méthode selon l'une quelconque des revendications 1 à 9, dans laquelle le ou les échantillons biologiques obtenus auprès d'un ou plusieurs patients après un arrêt cardiaque ont été obtenus dans les 96 heures, de préférence dans les 72 heures, mieux encore entre 24 et 72 heures, et de manière préférée entre toutes environ 48 heures après l'arrêt cardiaque.

11. Méthode selon l'une quelconque des revendications 1 à 10, comprenant en outre la prédiction de l'issue neurologique après un arrêt cardiaque chez le patient sur la base d'un ou plusieurs sélectionnés parmi l'âge du patient, le rythme choquable, la concentration d'hémoglobine, le temps entre l'arrêt cardiaque et le début du soutien vital, le temps entre la réanimation cardio-respiratoire et le retour à une circulation spontanée (ROSC), le lactate sérique maximal dans les 24 heures suivant l'arrêt cardiaque, la différence maximale avec le pH de 7,3 dans les 24 heures suivant l'arrêt cardiaque, et le niveau de la protéine S-110B dans l'échantillon du patient.

12. Utilisation d'un agent liant qui se lie spécifiquement à l'alpha-énolase dans une méthode pour prédire l'issue neurologique d'un patient après un arrêt cardiaque selon la revendication 1.

Figure 1

**299 biomarker candidates
in the sentinel cohort**
*Total number of identified proteins in plasma of
OHCA survivors 48 hours after hospital admission
by Shotgun LC-MS analysis
after depletion of top 12 abundant proteins*

**Biological relevance: 135 biomarker candidates**
*Crosschecking with proteins identified
in brain tissue samples
by Shotgun LC-MS analysis (n=4595). Only those proteins that
were detected in both datasets were included*

**Biomarker selection: 60 biomarker candidates**
*Proteins discerning favorable from poor neurological outcome
based on the following criteria:
Univariate performance, multivariate performance,
fold change of LFQ values >2*

**Technical validation: 24 biomarker candidates**
*Development of Targeted LC-MRM analysis based on
specificity, transition signals,
reproducibility, sensitivity.*

**Final selection of 24 biomarker candidates for
further verification in the verification cohort**
*ACTN1, APOE, C7, CAT, CDH13, CFL1, CLIC1, CNDP1, ENO1,
F13A1, FN1, GAPDH, HSPA8, ICAM1, LDHA, LDHB, PFN1, PGK1,
S100A8, S100A9, TLN1, VCL, VWF, YWHAZ*

Figure 2

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6080560 A
- US 4946778 A

- WO 2008022759 A


**Non-patent literature cited in the description**

- **WEAVER WD ; COBB LA ; HALLSTROM AP ; FAHRENBRUCH C ; COPASS MK ; RAY R.** Factors influencing survival after out-of-hospital cardiac arrest. *J Am Coll Cardiol,* 1986, vol. 7, 752-7
- **ATWOOD C ; EISENBERG MS ; HERLITZ J ; REA TD.** Incidence of EMS-treated out-of-hospital cardiac arrest in Europe. *Resuscitation,* 2005, vol. 67, 75-80
- **BENJAMIN EJ ; VIRANI SS ; CALLAWAY CW et al.** Heart Disease and Stroke Statistics-2018 Update: A Report From the American Heart Association. *Circulation,* 2018, vol. 137, e67-e492
- **GRASNER JT ; LEFERING R ; KOSTER RW et al.** EuReCa ONE-27 Nations, ONE Europe, ONE Registry: A prospective one month analysis of out-of-hospital cardiac arrest outcomes in 27 countries in Europe. *Resuscitation,* 2016, vol. 105, 188-95
- **REA TD ; EISENBERG MS ; SINIBALDI G ; WHITE RD.** Incidence of EMS-treated out-of-hospital cardiac arrest in the United States. *Resuscitation,* 2004, vol. 63, 17-24
- **LEMIALE V ; DUMAS F ; MONGARDON N et al.** Intensive care unit mortality after cardiac arrest: the relative contribution of shock and brain injury in a large cohort. *Intensive Care Med,* 2013, vol. 39, 1972-80
- **NOLAN JP ; SOAR J ; CARIOU A et al.** European Resuscitation Council and European Society of Intensive Care Medicine 2015 guidelines for post-resuscitation care. *Intensive Care Med,* 2015, vol. 41, 2039-56
- **WIJDICKS EF ; HIJDRA A ; YOUNG GB ; BASSETTI CL ; WIEBE S.** Practice parameter: prediction of outcome in comatose survivors after cardiopulmonary resuscitation (an evidence-based review): report of the Quality Standards Subcommittee of the American Academy of Neurology. *Neurology,* 2006, vol. 67, 203-10
- Guidelines 2000 for Cardiopulmonary Resuscitation and Emergency Cardiovascular Care. Part 2: ethical aspects of CPR and ECC. *Circulation,* 2000, vol. 102, 12-21

- **LAM MP ; PING P ; MURPHY E.** Proteomics Research in Cardiovascular Medicine and Biomarker Discovery. *J Am Coll Cardiol,* 2016, vol. 68, 2819-2830
- **DONNINO et al.** *Circulation,* 2015, vol. 132, 2448-2456
- **MAYER, BUKAU.** *Cell Mol Life Sci,* 2005, vol. 62 (6), 670-684
- **TAVARIA et al.** *Genomics,* 1995, vol. 29 (1), 266-268
- **AJAM.** *Scand J Trauma Resusc Emerg Med,* 2011, vol. 19, 38
- **WOJTCZAK-SOSKA ; LELONEK.** *Cardiol J,* 2010, vol. 17 (5), 532-6
- **LAITIO et al.** *JAMA,* 2016, vol. 315 (11), 1120-8
- **DEZFULIAN et al.** *Nitric oxide,* 2012, vol. 26 (4), 241-50
- **ARGAUD et al.** *JAMA Cardiol,* 2016, vol. 1 (5), 557-65
- **HAN et al.** *J Cell Mol Med,* 2017, vol. 22, 3167-3182
- **WANG et al.** *Stroke,* 2017, vol. 48 (8), 2211-2221
- **HUA et al.** *Neural Regen Res,* 2017, vol. 12 (1), 153-160
- **HAUSOTT ; KLIMASCHWESKI.** *Mol Neurobiol,* 17 September 2018
- **BASTIAN et al.** *Neurobiol Dis,* 2018
- **BEVERS et al.** *Exp Neurol.,* 2010, vol. 224 (1), 170-177
- **ESAU et al.** *JBC,* 2004, vol. 279, 52361-52365
- **GRIBBINGS et al.** *Nature Cell Biology,* 2009, vol. 11, 1143-1149
- **ELBASHIR et al.** *Nature,* 24 May 2001, vol. 411 (6836), 494-8
- **ZIMMERMANN et al.** *Nature,* 2006, vol. 441, 111-114
- **LO ; QI.** *F100Res,* 25 May 2017, vol. 6, 747
- **TAMHANE ; LOGAN.** Multiple Test Procedures for Identifying the Minimum Effective and Maximum Safe Doses of a Drug. *Journal of the American statistical association,* 2002, vol. 97 (457), 1-9

- **HARLOW ; LANE.** Antibodies, A Laboratory Manual. Cold Spring Harbor Laboratory Press, 198
- **HARLOW ; LANE.** Using Antibodies: A Laboratory Manual. Cold Spring Harbor Laboratory Press, 1999
- **ALTSHULER EP ; SEREBRYANAYA DV ; KATRUKHA AG.** *Biochemistry (Mosc),* 2010, vol. 75 (13), 1584
- **HOLLIGER P ; HUDSON PJ.** *Nat Biotechnol.,* 2005, vol. 23 (9), 1126
- **KONTERMANN ; BRINKMANN.** *Drug Discovery Today,* 2015, vol. 20 (7), 838-847
- **HARLOW E ; LANE D.** Using Antibodies: A Laboratory Manual. Cold Spring Harbor Laboratory Press, 1999
- **KOZBOR D.** *Immunology Today,* 1983, vol. 4, 7
- **LI J et al.** *PNAS,* 2006, vol. 103 (10), 3557
- **HOLLIGER P ; HUDSON PJ.** *Nat Biotechnol,* 2005, vol. 23 (9), 11265
- **FELDWISCH J ; TOLMACHEV V.** *Methods Mol Biol,* 2012, vol. 899, 103-26
- **GEBAUER ; SKERRA.** *Curr Opinion in Chemical Biology,* 2009, vol. 13, 245-255
- **BESTE G ; SCHMIDT FS ; STIBORA T ; SKERRA A.** *Proc Natl Acad Sci USA,* 1999, vol. 96 (5), 1898-903
- **WEIDLE UH et al.** *Cancer Genomics Proteomics,* 2013, vol. 10 (4), 155-68
- **MOURATOU B ; BÉHAR G ; PAILLARD-LAURANCE L ; COLINET S ; PECORARI F.** *Methods Mol Biol.,* 2012, vol. 805, 315-31
- **WEIDLE et al.** *Cancer Genomics Proteomics,* 2013, vol. 10 (4), 155-68
- **GRABULOVSKI et al.** *JBC,* 2007, vol. 282, 3196-3204
- **BERTSCHINGER et al.** *Protein Eng Des Sel,* 2007, vol. 20 (2), 57-68
- **SCHLATTER et al.** *MAbs,* 2012, vol. 4 (4), 1-12
- **OSBORNE.** *Current Opinion in Chemical Biology,* 1997, vol. 1, 5-9
- **STULL ; SZOKA.** *Pharmaceutical Research,* 1995, vol. 12 (4), 465-483
- **SAMBROOK et al.** Molecular Cloning: A Laboratory Manual. Cold Spring Harbor Laboratory Press, 1989
- **AUSUBEL et al.** Current Protocols in Molecular Biology. Greene Publishing Associates, 1992
- Harlow and Lane Antibodies: A Laboratory Manual. Cold Spring Harbor Laboratory Press, 1990
- **URBANIAK et al.** *Research Randomizer,* 2013
- **CALLAWAY et al.** *Circulation,* 2015, vol. 132 (16), S84-145
- **JACOBS et al.** *Circulation,* 2004, vol. 110 (21), 3385-97
- **MUQAKU et al.** *Proteomics,* 2017, vol. 16 (1), 86-99
- **COX et al.** *Nature biotechnology,* 2008, vol. 26 (12), 1367-72
- **CRONBERG et al.** *Neurology,* 2011, vol. 77 (7), 623-30
- **HOERL et al.** *Technometrics,* 1970, vol. 12 (1), 55-67
- **ZOU et al.** *Journal of the Royal Statistical Society Series B,* 2005, vol. 67 (2), 301-20